# EUROPEAN PATENT APPLICATION

(11) **EP 4 834 877 A2**
(43) Date of publication of application: **04.11.2026**
(21) Application number: 26205887.8
(22) Date of filing: 01.09.2022
(51) Int. Cl.: C07C 227/18

(54) **SYNTHESIS OF NIROGACESTAT**

(30) Priority: 01.09.2021 US 202163239696 P
(62) Document number(s) of the earlier application(s) in accordance with Art. 76 EPC: 22865803.5 / 4 396 159
(71) Applicant: SpringWorks Therapeutics, Inc., Stamford, CT 06902 (US)
(72) Inventor: PATTERSON, Kristin, Stamford, 06902 (US); HATCHER, Mark, Stamford, 06902 (US)
(74) Representative: Nederlandsch Octrooibureau

(57) **Abstract**

The present disclosures are directed to processes for synthesizing (S)-2-(((S)-6,8-difluoro-1,2,3,4-tetrahydronaphthalen-2-yl)amino)-N-(1-(2-methyl-1-(neopentylamino)propan-2-yl)-1H-imidazol-4-yl)pentanamide ("nirogacestat").

## Description

### FIELD OF THE DISCLOSURE

The present disclosure relates to processes of synthesizing (S)-2-(((S)-6,8-difluoro-1,2,3,4-tetrahydronaphthalen-2-yl)amino)-N-(1-(2-methyl-1-(neopentylamino)propan-2-yl)-1H-imidazol-4-yl)pentanamide ("nirogacestat" or "Compound 1").

### BACKGROUND

(S)-2-(((S)-6,8-difluoro-1,2,3,4-tetrahydronaphthalen-2-yl)amino)-N-(1-(2-methyl-1-(neopentylamino)propan-2-yl)-1H-imidazol-4-yl)pentanamide ("nirogacestat" or "Compound 1") exhibits promising activity for the treatment of tumors or cancer, such as desmoid tumors, multiple myeloma, a cancer having a mutation in a Notch pathway gene, adenoid cystic carcinoma, and T-cell acute lymphoblastic leukemia (U.S. Patent No. 10,590,087). A known route of synthesizing nirogacestat offers few points for the control of impurities other than at the final isolation (Bioorganic & Medicinal Chemistry Letters 21:2637-2640 (2011)). If control of impurities is not optimal in the late stage intermediates, impurities are likely to be present in the product at reportable levels. This route also exhibits a low yield when a norvaline moiety and tetralone fragments are coupled (Bioorganic & Medicinal Chemistry Letters 21:2637-2640 (2011)). Therefore, there exists a need for developing a new route to introduce additional control points to purge impurities and minimize any loss of stereochemical integrity.

### BRIEF SUMMARY OF THE DISCLOSURE

Processes comprising reacting a novaline moiety with 1,1'-carbonyldiimidazole (CDI) to form an activated anhydride are provided herein. For example, processes comprising reacting Compound **9** 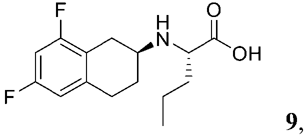 with 1,1'-carbonyldiimidazole under conditions suitable to form Compound **10** 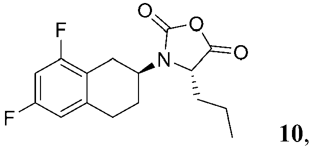 or a pharmaceutically acceptable salt thereof, are described herein. In some aspects, the process of forming Compound **10,** or a pharmaceutically acceptable salt thereof, occurs in the presence of a polar aprotic solvent. In some aspects, the polar aprotic solvent is dimethyl sulfoxide (DMSO), N,N-dimethylformamide (DMF), acetone, dichloromethane, tetrahydrofuran (THF), N-methyl-2-pyrrolidone (NMP), 1,4-dioxane, acetonitrile, or a combination thereof. In some aspects, the process of forming Compound **10,** or a pharmaceutically acceptable salt thereof, occurs in the presence of one or more additives. In some aspects, the one or more additives comprise pyridine hydrobromide. In some aspects, the one or more additives comprise triethylamine. In some aspects, Compound **10,** or a pharmaceutically acceptable salt thereof, is used in a subsequent process without isolation or purification.

Processes comprising reacting Compound **10** with Compound **11** 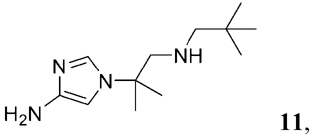 under conditions suitable to form Compound **1** 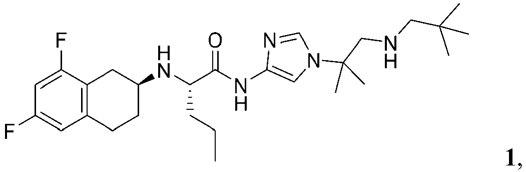 or a pharmaceutically acceptable salt thereof, are also described herein. In some aspects, the process of reacting Compound **10** with Compound **11** occurs in the presence of a polar aprotic solvent. In some aspects, the polar aprotic solvent is dimethyl sulfoxide (DMSO), N,N-dimethylformamide (DMF), acetone, tetrahydrofuran (THF), N-methyl-2-pyrrolidone (NMP), acetonitrile, or a combination thereof. In some aspects, Compound **11** is prepared without isolation or purification, and reacted with Compound **10.** In some aspects, Compound **1** is neither purified nor isolated.

In some aspects, Compound **1** is the free base. In some aspects, Compound **1** is a pharmaceutically acceptable salt. In some aspects, Compound **1** is the dihydrobromide salt. In some aspects, the dihydrobromide salt of Compound **1** is a crystalline solid.

In some aspects, the process further comprises reacting Compound **1** free base with an aqueous inorganic acid under conditions suitable to form a pharmaceutically acceptable salt of Compound **1.** In some aspects, the pH is adjusted to about 1 to about 1.5. In some aspects, the inorganic acid is hydrobromic acid.

In some aspects, the pharmaceutically acceptable salt of Compound **1** is isolated. In some aspects, the pharmaceutically acceptable salt of Compound **1** is the hydrobromide salt. In some aspects, the pharmaceutically acceptable salt of Compound **1** is the dihydrobromide salt.

Processes comprising reacting Compound **12** 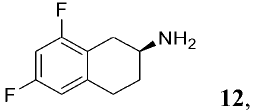 with a compound of Formula **IV** 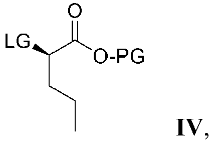 under conditions suitable to form a compound of Formula **V** 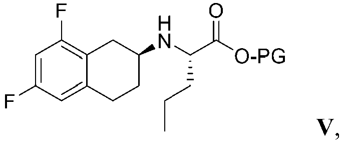 or a pharmaceutically acceptable salt thereof, are also described herein, wherein LG is a leaving group and PG is a protecting group. In some aspects, LG is -OR², R² is - S(=O)₂R³, and R³ is C₁-C₃ alkyl, C₁-C₃ haloalkyl, or optionally substituted phenyl. In some aspects, R² is -S(=O)₂CF₃. In some aspects, PG is a C₁-C₆ alkyl. In some aspects, PG is t-butyl. In some aspects, the process of forming a compound of Formula **V,** or a pharmaceutically acceptable salt thereof, occurs in the presence of a polar aprotic solvent. In some aspects, the polar aprotic solvent is dimethyl sulfoxide (DMSO), N,N-dimethylformamide (DMF), acetone, dichloromethane, tetrahydrofuran (THF), N-methyl-2-pyrrolidone (NMP), 1,4-dioxane, acetonitrile, or a combination thereof.

In some aspects, the process of forming a compound of Formula IV, or a pharmaceutically acceptable salt thereof, occurs in the presence of a base. In some aspects, the base is N,N-diisopropylethylamine. In some aspects, the solvent is removed *in vacuo.*

In some aspects, the compound of Formula **V** is dissolved in a polar aprotic solvent and an aqueous inorganic acid. In some aspects, the polar aprotic solvent is dimethyl sulfoxide (DMSO), N,N-dimethylformamide (DMF), acetone, dichloromethane, tetrahydrofuran (THF), N-methyl-2-pyrrolidone (NMP), 1,4-dioxane, acetonitrile, or a combination thereof. In some aspects, a pharmaceutically acceptable salt of Formula **V** is isolated. In some aspects, the pharmaceutically acceptable salt of Formula **V** is isolated by filtration. In some aspects, the inorganic acid is hydrochloric acid. In some aspects, the pharmaceutically acceptable salt of Formula **V** is the hydrochloride salt.

Processes further comprising reacting a compound of Formula **V,** or pharmaceutically acceptable salt of thereof, with an aqueous inorganic acid in a polar protic solvent, under conditions suitable to form Compound **9** 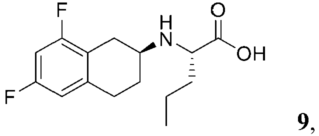 or a pharmaceutically acceptable salt thereof, are also described herein. In some aspects, in the process in which a compound of Formula **V,** or pharmaceutically acceptable salt thereof, is reacted with an aqueous inorganic acid, the polar protic solvent is an alcohol. In some aspects, the alcohol is isopropanol.

In some aspects, in the process in which a compound of Formula **V,** or pharmaceutically acceptable thereof, is reacted with an aqueous inorganic acid, the pH is adjusted to about 2.6 to about 3.0.

In some aspects, in the process in which a compound of Formula **V,** or pharmaceutically acceptable thereof, is reacted with an aqueous inorganic acid, Compound **9,** or pharmaceutically acceptable salt thereof, is isolated. In some aspects, Compound **9,** or pharmaceutically acceptable salt thereof, is isolated by filtration.

In some aspects, in the process in which a compound of Formula **V,** or pharmaceutically acceptable thereof, is reacted with an aqueous inorganic acid, the inorganic acid is hydrochloric acid.

The disclosure further relates to processes of preparing Compound **1,** or a pharmaceutically acceptable salt thereof, by reacting Compound **10,** or pharmaceutically acceptable salt thereof, with Compound **11,** or pharmaceutically acceptable salt thereof. In some aspects, the process of preparing Compound 1, or a pharmaceutically acceptable salt thereof, occurs in the presence of a polar aprotic solvent. In some aspects, the polar aprotic solvent is dimethyl sulfoxide (DMSO), N,N-dimethylformamide (DMF), acetone, tetrahydrofuran (THF), N methyl-2-pyrrolidone (NMP), acetonitrile, or a combination thereof.

Processes comprising reacting Compound **1** free base, prepared by any of the processes described above, with an aqueous inorganic acid under conditions suitable to form a pharmaceutically acceptable salt of Compound **1** are provided herein. In some aspects, the pH of the process of Compound **1** free base with an aqueous inorganic acid is about 1 to about 1.5. In some aspects, the inorganic acid is hydrobromic acid. In some aspects, the pharmaceutically acceptable salt of Compound **1** is isolated. In some aspects, the pharmaceutically acceptable salt of Compound **1** is the hydrobromide salt. In some aspects, the pharmaceutically acceptable salt of Compound **1** is the dihydrobromide salt.

The disclosure further relates to processes comprising reacting Compound **9** in a solvent with 1,1'-carbonyldiimidazole under conditions suitable to form Compound **10,** wherein the process further comprises reacting Compound **10** with Compound **11** under conditions suitable to form Compound **1,** or a pharmaceutically acceptable salt thereof. In some aspects, the process of forming Compound **1,** or a pharmaceutically acceptable salt thereof, occurs in the presence of a polar aprotic solvent. In some aspects, the polar aprotic solvent is dimethyl sulfoxide (DMSO), N,N-dimethylformamide (DMF), acetone, dichloromethane, tetrahydrofuran (THF), N-methyl-2-pyrrolidone (NMP), 1,4-dioxane, acetonitrile, or a combination thereof. In some aspects, the process of forming Compound **1,** or a pharmaceutically acceptable salt thereof, occurs in the presence of one or more additives. In some aspects, the one or more additives comprise pyridine hydrobromide. In some aspects, the one or more additives comprise triethylamine.

In some aspects, the process further comprises reacting Compound **1** free base with an aqueous inorganic acid under conditions suitable to form a pharmaceutically acceptable salt of Compound **1.** In some aspects, the pH is adjusted to about 1 to about 1.5. In some aspects, the aqueous inorganic acid is hydrobromic acid.

The disclosure further relates to processes comprising reacting a pharmaceutically acceptable salt of Compound **1** with an aqueous inorganic acid in an alcohol. In some aspects, the alcohol is isopropanol. In some aspects, the pH is adjusted to about 3 to about 3.5. In some aspects, a pharmaceutically acceptable salt of Compound **1** is isolated. In some aspects, the pharmaceutically acceptable salt of Compound **1** is isolated by filtration. In some aspects, the aqueous inorganic acid is hydrobromic acid.

In some aspects, Compound **1** is synthesized by any one of the processes described above. In some aspects, the pharmaceutically acceptable salt of Compound **1** of the processes described herein is isolated. In some aspects, the pharmaceutically acceptable salt of Compound **1** is the hydrobromide salt. In some aspects, the pharmaceutically acceptable salt of Compound **1** is the dihydrobromide salt.

The disclosure further relates to compositions comprising 98.0% to 99.9% of a Compound **1,** or a pharmaceutically acceptable salt thereof, and one or more of the following:
(1) 0.7% to 0.01% of a compound of Formula **II:** 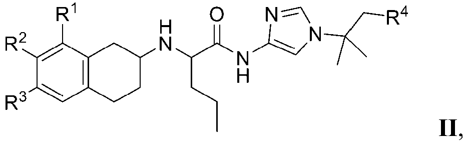 wherein:
   R¹ is selected from the group consisting of hydrogen, fluoro, or chloro;
   R² is selected from the group consisting of hydrogen or chloro;
   R³ is selected from the group consisting of fluoro, or chloro; and
   R⁴ is selected from the group consisting of -OH and -N(H)CH₂C(CH₃)₃,
   or a pharmaceutically acceptable salt thereof;
(2) 0.05% to 0.005% of imidazole, or a pharmaceutically acceptable salt thereof; or
(3) 0.7% to 0.01% of a compound of Formula **II,** or a pharmaceutically acceptable salt thereof, and 0.05% to 0.005% of imidazole, or a pharmaceutically acceptable salt thereof, wherein the compound of Formula **II** is not Compound **1,** and the percentages of Compound **1,** the compound of Formula **II,** and/or imidazole are determined by high performance liquid chromatography. In some aspects, the compound of Formula **II** is a hydrobromide salt. In some aspects, Compound **1** is a hydrobromide salt. In some aspects, Compound **1** is a dihydrobromide salt.

In some aspects, the compound of Formula **II** is a compound of Formula **II-A** 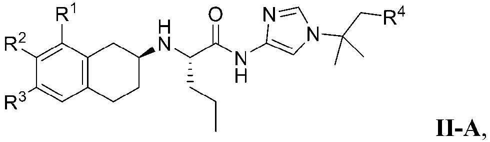 or a pharmaceutically acceptable salt thereof. In some aspects, the compound of Formula **II-A** is a hydrobromide salt. In some aspects, the compound of Formula **II-A** is a dihydrobromide salt.

In some aspects, the compound of Formula **II** is a compound of Formula **II-B** 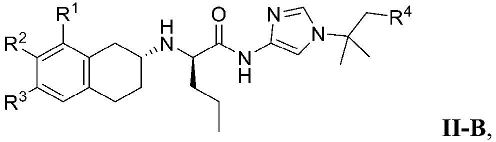 or a pharmaceutically acceptable salt thereof. In some aspects, the compound of Formula **II-B** is a hydrobromide salt. In some aspects, the compound of Formula **II-B** is a dihydrobromide salt.

In some aspects, the compound of Formula **II** is a compound of Formula **II-C:** 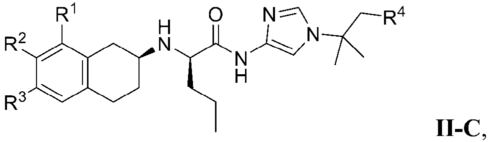 or a pharmaceutically acceptable salt thereof. In some aspects, the compound of Formula **II-C** is a hydrobromide salt. In some aspects, the compound of Formula **II-C** is a dihydrobromide salt.

In some aspects, the compound of Formula **II** is a compound of Formula **II-D** 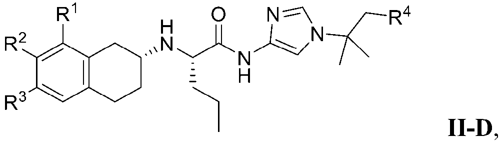 or a pharmaceutically acceptable salt thereof. In some aspects, the compound of Formula **II-D** is a hydrobromide salt. In some aspects, the compound of Formula **II-D** is a dihydrobromide salt.

In some aspects, R⁴ is -N(H)CH₂C(CH₃)₃. In some aspects, R⁴ is -OH. In some aspects, R¹ is fluoro, R² is hydrogen, and R³ is chloro. In some aspects, R¹ is chloro, R² is hydrogen, and R³ is fluoro. In some aspects, R¹ is hydrogen, R² is chloro, and R³ is fluoro. In some aspects, R¹ is fluoro, R² is hydrogen, and R³ is fluoro.

The disclosure further relates to compositions comprising 98.0% to 99.9% of Compound **1,** or a pharmaceutically acceptable salt thereof, and 0.6% to 0.01% of Compound **2** 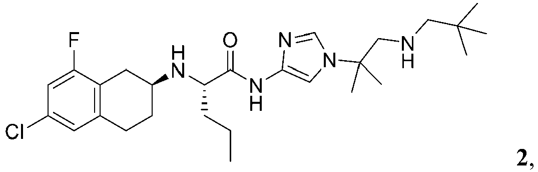 or a pharmaceutically acceptable salt thereof. In some aspects, Compound **2** is a hydrobromide salt. In some aspects, Compound **2** is a dihydrobromide salt. In some aspects, Compound **1** is a hydrobromide salt. In some aspects, Compound **1** is a dihydrobromide salt.

The disclosure further relates to compositions comprising 98.0% to 99.9% of Compound **1,** or a pharmaceutically acceptable salt thereof, and 0.3% to 0.01% of Compound **3** 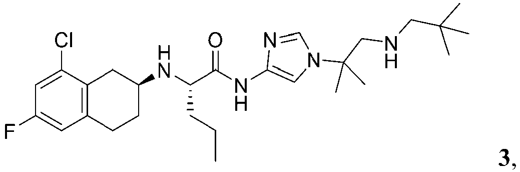 or a pharmaceutically acceptable salt thereof. In some aspects, Compound **3** is a hydrobromide salt. In some aspects, Compound **3** is a dihydrobromide salt. In some aspects, Compound **1** is a hydrobromide salt. In some aspects, Compound **1** is a dihydrobromide salt.

The disclosure further relates to compositions comprising 98.0% to 99.9% of Compound **1,** or a pharmaceutically acceptable salt thereof, and 0.5% to 0.01% of Compound **4** 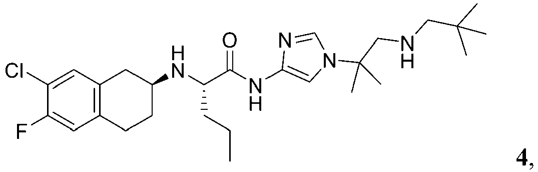 or a pharmaceutically acceptable salt thereof. In some aspects, Compound **4** is a hydrobromide salt. In some aspects, Compound **4** is a dihydrobromide salt. In some aspects, Compound **1** is a hydrobromide salt. In some aspects, Compound **1** is a dihydrobromide salt.

The disclosure further relates to compositions comprising 98.0% to 99.9% of Compound **1,** or a pharmaceutically acceptable salt thereof, and 0.5% to 0.01% of Compound **5** 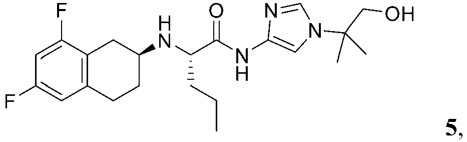 or a pharmaceutically acceptable salt thereof. In some aspects, Compound **5** is a hydrobromide salt. In some aspects, Compound **5** is a dihydrobromide salt. In some aspects, Compound **1** is a hydrobromide salt. In some aspects, Compound **1** is a dihydrobromide salt.

The disclosure further relates to compositions comprising 98.0% to 99.9% of Compound **1,** or a pharmaceutically acceptable salt thereof, and 0.5% to 0.01% of Compound **6** 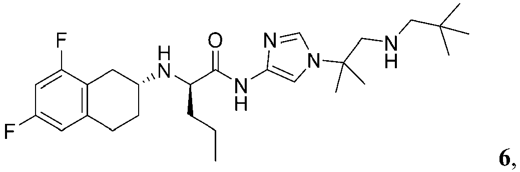 or a pharmaceutically acceptable salt thereof. In some aspects, Compound **6** is a hydrobromide salt. In some aspects, Compound **6** is a dihydrobromide salt. In some aspects, Compound **1** is a hydrobromide salt. In some aspects, Compound **1** is a dihydrobromide salt.

The disclosure further relates to compositions comprising 98.0% to 99.9% of Compound **1,** or a pharmaceutically acceptable salt thereof, and 0.5% to 0.01% of Compound **7** 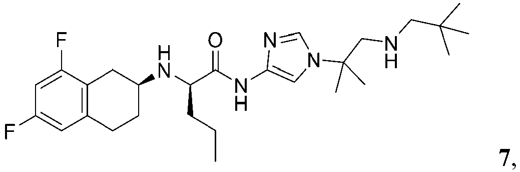 or a pharmaceutically acceptable salt thereof. In some aspects, Compound **7** is a hydrobromide salt. In some aspects, Compound **7** is a dihydrobromide salt. In some aspects, Compound **1** is a hydrobromide salt. In some aspects, Compound **1** is a dihydrobromide salt.

The disclosure further relates to compositions comprising 98.0% to 99.9% of Compound **1,** or a pharmaceutically acceptable salt thereof, and 0.5% to 0.01% of Compound **8** 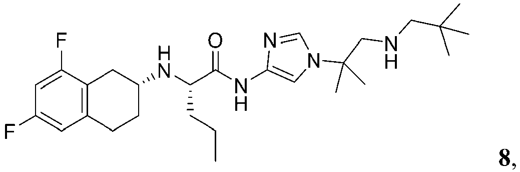 or a pharmaceutically acceptable salt thereof. In some aspects, Compound **8** is a hydrobromide salt. In some aspects, Compound **8** is a dihydrobromide salt. In some aspects, Compound **1** is a hydrobromide salt. In some aspects, Compound **1** is a dihydrobromide salt.

The disclosure further relates to compositions comprising 98.0% to 99.9% of Compound **1,** or a pharmaceutically acceptable salt thereof, and 0.05% to 0.005% of imidazole, or a pharmaceutically acceptable salt thereof. In some aspects, the imidazole is a hydrobromide salt. In some aspects, Compound **1** is a hydrobromide salt. In some aspects, Compound **1** is a dihydrobromide salt.

The disclosure further relates to processes for preparing a composition with an active agent susceptible to oxidation that is substantially free of Compound **13** 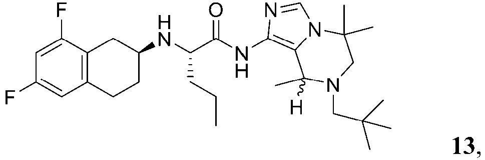 comprising the steps of dissolving Compound **11** in a solvent and combining the solution of Compound **11** with Compound **9** to form a mixture that is treated with hydrobromic acid. In some aspects, the active agent susceptible to oxidation is a hydrobromide salt of Compound **1.** In some aspects, the active agent susceptible to oxidation is a dihydrobromide salt of Compound **1.**

In some aspects, the solvent used to dissolve Compound **11** is an alcohol of the formula R-OH, wherein R is an alkyl, with the proviso that R is not ethyl. In some aspects, the solvent is a primary alcohol other than ethanol. In some aspects, the solvent is a secondary alcohol. In some aspects, the solvent is a tertiary alcohol. In some aspects, the solvent is 2-methylpropan-1-ol. In some aspects, the solvent is propanol. In some aspects, the solvent is isopropanol. In some aspects, the mixture is warmed to 0-5 °C during hydrobromic acid treatment.

In some aspects, the mixture is maintained at a pH less than 6. In some aspects, the mixture is maintained at a pH less than 3.

The disclosure further relates to processes for preparing a composition of Compound **1,** which is essentially free of Compound **13,** comprising the steps of combining Compound **10** with Compound **11** to form a mixture that is treated with hydrobromic acid in a solvent. In some aspects, the composition of Compound **1** is a hydrobromide salt. In some aspects, the composition of Compound **1** is a dihydrobromide salt.

In some aspects, Compound **1** is synthesized by any one of the processes described above.

### DETAILED DESCRIPTION

### Definitions and Abbreviations

As used above, and throughout the description, the following terms, unless otherwise indicated, shall be understood to have the following meanings.

Unless stated otherwise, the terms "a" and "an" and "the" and similar references used in the context of describing a particular aspect of the application (especially in the context of claims) can be construed to cover both the singular and the plural. The recitation of ranges of values herein is merely intended to serve as a shorthand process of referring individually to each separate value falling within the range. Unless otherwise indicated herein, each individual value is incorporated into the specification as if it were individually recited herein.

The term "alkyl" means any monovalent, saturated straight, branched or cyclic hydrocarbon group. Examples of "alkyl" groups include methyl, ethyl, isopropyl, and the like.

The term "haloalkyl" as used by itself or as part of another group refers to an alkyl group substituted by one or more fluorine, chlorine, bromine and/or iodine atoms.

The term "substituted" refers to independent replacement of one or more hydrogen atoms on the substituted moiety with substituents independently selected from the group of substituents as specified for a particular group. In general, a non-hydrogen substituent can be any substituent that can be bound to an atom of the given moiety that is specified to be substituted.

The term "leaving group" as used herein refers to group that departs with a pair of electrons in heterolytic bond cleavage. Common leaving groups are halides such as Cl⁻, Br⁻, and I⁻, and sulfonate esters such as tosylate (TsO⁻).

The term "protecting group" as used herein refers to group that blocks, i.e., protects, the amine functionality while reactions are carried out on other functional groups or parts of the molecule. Those skilled in the art will be familiar with the selection, attachment, and cleavage of protecting groups and will appreciate that different protective groups are known in the art, the suitability of one protective group or another being dependent on the particular the synthetic scheme planned. Treatises on the subject are available for consultation, such as Wuts, "Greene's Protective Groups in Organic Synthesis", 5th Ed., J. Wiley & Sons, Inc., NY, 2014. Suitable protecting groups include carbobenzyloxy (Cbz), *tert*-butyloxycarbonyl (BOC), 9-fluorenylmethyloxycarbonyl (FMOC) and benzyl (Bn) groups.

The term "base" as used herein refers to an organic proton acceptor. Non limiting bases include non-nucleophilic tertiary amines, e.g., NEt₃, N,N-Diisopropylethylamine, and nitrogen-containing heteroaromatic groups such as pyridine, and derivatives of pyridine, e.g., 2,4,6-trimethylpyridine.

The terms "isolate," "isolated," "isolation," and "isolating" as used herein mean a substance remains alone or apart from a solution.

The terms "purify," "purified," "purification," and "purifying" as used herein mean the removal of contaminants from a substance of interest, *e.g.,* Compound **1.**

### Nirogacestat Synthesis

Processes comprising reacting a novaline moiety with 1,1'-carbonyldiimidazole (CDI) to form an activated anhydride are described herein. The process can then be followed by coupling the activated anhydride with an imidazole amine to afford Nirogacestat, or a pharmaceutically acceptable salt thereof (*e.g.,* dihydrobromide). This coupling reaction minimizes any loss of stereochemical integrity of the norvaline moiety through buffering CDI with pyridine hydrobromide. All steps avoid aqueous workups and distillations while enabling facile crystallizations. The isolation of Nirogacestat, or pharmaceutically acceptable salt thereof (*e.g.,* dihydrobromide) is well-designed, providing crystalline material in high yield and purity.

The disclosure also relates to processes of preparing Compound **1,** or a pharmaceutically acceptable salt thereof (*e.g.,* dihydrobromide), comprising reacting a novaline moiety directly with an imidazole amine in the presence of an alcohol other than ethanol to yield nirogacestat. This process limits the impurity in the final product of nirogacestat.

Processes comprising reacting Compound **9** 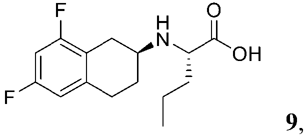 with 1,1'-carbonyldiimidazole under conditions suitable to form Compound **10** 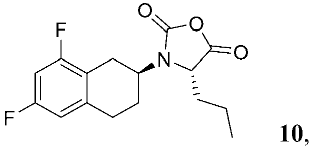 or a pharmaceutically acceptable salt thereof, are described herein. In some aspects, the 1,1'-carbonyldiimidazole is present in the process in an amount of about 1 to about 2 equivalents of Compound **9.** In some aspects, the 1,1'-carbonyldiimidazole is present in the process in an amount of about 1.1 equivalents of Compound **9.**

In some aspects, the process of forming Compound **10,** or a pharmaceutically acceptable salt thereof, occurs in the presence of a polar aprotic solvent. In some aspects, the polar aprotic solvent is dimethyl sulfoxide (DMSO), N,N-dimethylformamide (DMF), acetone, dichloromethane, tetrahydrofuran (THF), N-methyl-2-pyrrolidone (NMP), 1,4-dioxane, acetonitrile, or a combination thereof. In some aspects, the polar aprotic solvent is acetonitrile.

In some aspects, the process of forming Compound **10,** or a pharmaceutically acceptable salt thereof, occurs in the presence of one or more additives. In some aspects, the one or more additives comprises pyridine hydrobromide. In some aspects, the pyridine hydrobromide is present in the process in an amount of about 2 to about 3 equivalents of Compound **9.** In some aspects, the pyridine hydrobromide is present in the process in an amount of about 2.1 equivalents of Compound **9.** In some aspects, the one or more additives comprises triethylamine. In some aspects, the triethylamine is present in the process in an amount of about 0.1 to 1 equivalents of Compound **9.** In some aspects, the triethylamine is present in the process in an amount of about 0.7 equivalents of Compound **9.**

In some aspects, the process of preparing Compound **10,** or a pharmaceutically acceptable salt thereof, occurs at a temperature from about 20 °C to about 30 °C. In some aspects, the process of preparing Compound **10,** or a pharmaceutically acceptable salt thereof, takes about four hours.

In some aspects, the process of forming Compound **10,** or a pharmaceutically acceptable salt thereof, is sampled for HPLC analysis. In some aspects, the process of forming Compound **10,** or a pharmaceutically acceptable salt thereof, stops until HPLC area% of Compound **9** is less than 10.0 area%. In some aspects, the process of forming Compound **10,** or a pharmaceutically acceptable salt thereof, stops until HPLC area% of Compound 9 is less than 2.0 area%. In some aspects, the difference between two consecutive Compound **9** HPLC analysis is less than 0.5 area%. In some aspects, the process of forming Compound **10,** or a pharmaceutically acceptable salt thereof, is cooled to a temperature from about -15 °C to about -5 °C. In some aspects, the process of forming Compound **10,** or a pharmaceutically acceptable salt thereof, is cooled to a temperature of about -7 °C. In some aspects, Compound **10** is used in a subsequent process without isolation or purification.

The disclosure relates to processes comprising reacting a pharmaceutically acceptable salt of Compound **11** 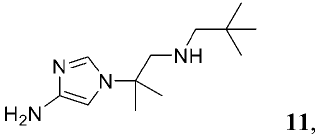 with a base in the presence of a polar aprotic solvent. In some aspects, the pharmaceutically acceptable salt of Compound **11** is a hydrobromide salt. In some aspects, the pharmaceutically acceptable salt of Compound **11** is a dihydrobromide salt. In some aspects, Compound **11** is a free base. In some aspects, the base is selected from the group consisting of trimethylamine, triethylamine, N,N-Diisopropylethylamine, and pyridine. In some aspects, the base is triethylamine. In some aspects, the triethylamine is present in the process in an amount of about 2 to about 5 equivalents of Compound **11.** In some aspects, the triethylamine is present in the process in an amount of about 3.1 equivalents of Compound **11.** In some aspects, the polar aprotic solvent is dimethyl sulfoxide (DMSO), N,N-dimethylformamide (DMF), acetone, dichloromethane, tetrahydrofuran (THF), N-methyl-2-pyrrolidone (NMP), 1,4-dioxane, acetonitrile, or a combination thereof. In some aspects, the polar aprotic solvent is acetonitrile. In some aspects, the process of preparing Compound **11,** or a free base thereof, occurs at a temperature from about -15 °C to about -5 °C. In some aspects, the process of preparing Compound **11,** or a free base thereof, occurs at a temperature of about -7 °C. In some aspects, Compound **11** is used in a subsequent process without isolation or purification.

Processes comprising reacting Compound **10** with Compound **11** under conditions suitable to form Compound **1** 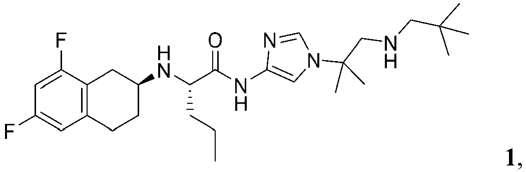 or a pharmaceutically acceptable salt thereof are also described herein. In some aspects, Compound **11** is a free base. In some aspects, Compound **11** is present in the process in an amount of about 1 to about 2 equivalents of Compound **10.** In some aspects, Compound **11** is present in the process in an amount of about 1.2 equivalents of Compound **10.** In some aspects, the process of reacting Compound **10** with Compound **11** occurs in the presence of a polar aprotic solvent. In some aspects, the polar aprotic solvent is dimethyl sulfoxide (DMSO), N,N-dimethylformamide (DMF), acetone, dichloromethane, tetrahydrofuran (THF), N-methyl-2-pyrrolidone (NMP), 1,4-dioxane, acetonitrile, or a combination thereof. In some aspects, the polar aprotic solvent is acetonitrile. In some aspects, the process of reacting Compound **10** with Compound **11** occurs at a temperature from about -15 °C to about 0 °C. In some aspects, the process of reacting Compound **10** with Compound **11** occurs at a temperature of about -7 °C. In some aspects, the process of reacting Compound **10** with Compound **11** takes about six hours. In some aspects, the process of reacting Compound **10** with Compound **11** is sampled for HPLC analysis. In some aspects, the process of reacting Compound **10** with Compound **11** stops until HPLC area% of Compound **10** is less than 5.0 area%. In some aspects, the process of reacting Compound **10** with Compound **11** stops until HPLC area% of Compound **10** is less than 1.0 area%. In some aspects, the difference between two consecutive Compound **10** HPLC analysis is less than 0.5 area%. In some aspects, the pharmaceutically acceptable salt of Compound **1** is a hydrobromide salt. In some aspects, the pharmaceutically acceptable salt of Compound **1** is a dihydrobromide salt. In some aspects, Compound **1** is a free base. In some aspects, Compound **1** is neither purified nor isolated.

The process further comprises reacting Compound **1** free base with an aqueous inorganic acid under conditions suitable to form a pharmaceutically acceptable salt of Compound **1.** In some aspects, the inorganic acid is hydrobromic acid. In some aspects, hydrobromic acid is present in the process in an amount of about 5 to about 10 equivalents of Compound **10.** In some aspects, hydrobromic acid is present in the process in an amount of about 7.5 equivalents of Compound **10.**

In some aspects, the process of forming a pharmaceutically acceptable salt of Compound **1** occurs at a temperature from about 30 °C to about 50 °C. In some aspects, the process of forming a pharmaceutically acceptable salt of Compound **1** occurs at a temperature of about 40 °C. In some aspects, the process of forming a pharmaceutically acceptable salt of Compound **1** occurs in the presence of one or more additives. In some aspects, the one or more additives comprise triethylamine. In some aspects, the pH of the process of forming a pharmaceutically acceptable salt of Compound **1** is adjusted to about 1.0 to about 1.5. In some aspects, the seeds of a pharmaceutically acceptable salt of Compound **1** is added. In some aspects, the amount of the seeds is about 0.1 wt%. In some aspects, the process of forming a pharmaceutically acceptable salt of Compound **1** is cooled at a temperature from about -15 °C to about 0 °C. In some aspects, the process of forming a pharmaceutically acceptable salt of Compound **1** is cooled at a temperature of about -5 °C. In some aspects, the pharmaceutically acceptable salt of Compound **1** is isolated. In some aspects, the pharmaceutically acceptable salt of Compound **1** is isolated as a crystalline solid. In some aspects, the pharmaceutically acceptable salt of Compound **1** is a hydrobromide salt. In some aspects, the pharmaceutically acceptable salt of Compound **1** is a dihydrobromide salt.

Processes comprising reacting Compound **12** 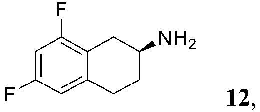 with a compound of Formula **IV** 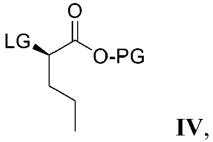 under conditions to form a compound of Formula **V** 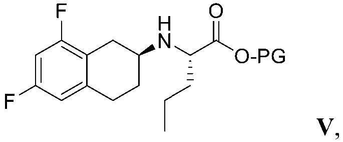 or a pharmaceutically acceptable salt thereof are also described herein, wherein LG is a leaving group; and PG is a protecting group. In some aspects, LG is -OR²; R² is - S(=O)₂R³; and R³ is C₁-C₃ alkyl, C₁-C₃ haloalkyl, or optionally substituted phenyl. In some aspects, R² is -S(=O)₂CF₃. In some aspects, PG is a C₁-C₆ alkyl. In some aspects, PG is t-butyl. In some aspects, the process of forming a compound of Formula **V,** or a pharmaceutically acceptable salt thereof, occurs in the presence of a polar aprotic solvent. In some aspects, the polar aprotic solvent is dichloromethane, dimethyl sulfoxide (DMSO), N,N-dimethylformamide (DMF), acetone, dichloromethane, tetrahydrofuran (THF), N-methyl-2-pyrrolidone (NMP), 1,4-dioxane, acetonitrile, or a combination thereof. In some aspects, the polar aprotic solvent is dichloromethane. In some aspects, the process of forming a compound of Formula **V,** or a pharmaceutically acceptable salt thereof, occurs at a temperature from about 20 °C to about 30 °C. In some aspects, the process of forming a compound of Formula **V,** or a pharmaceutically acceptable salt thereof, occurs at a temperature of about 30 °C. In some aspects, the process of forming a compound of Formula **V,** or a pharmaceutically acceptable salt thereof, takes about ten hours. In some aspects, the process of forming a compound of Formula **V,** or a pharmaceutically acceptable salt thereof, is sampled for HPLC analysis. In some aspects, the process of forming a compound of Formula **V,** or a pharmaceutically acceptable salt thereof, stops until HPLC area% of Compound **12** is less than 2.0 area%. In some aspects, the process of forming a compound of Formula **V,** or a pharmaceutically acceptable salt thereof, stops until HPLC area% of Compound **12** is less than 1.0 area%. In some aspects, the difference between two consecutive Compound **12** HPLC analysis is less than 0.5 area%.

In some aspects, the process of forming a compound of Formula **IV,** or a pharmaceutically acceptable salt thereof, occurs in the presence of a base. In some aspects, the base is N,N-diisopropylethylamine. In some aspects, the solvent is removed *in vacuo.*

In some aspects, the compound of Formula **V** is dissolved in a polar aprotic solvent and an aqueous inorganic acid. In some aspects, the polar aprotic solvent is dimethyl sulfoxide (DMSO), N,N-dimethylformamide (DMF), acetone, dichloromethane, tetrahydrofuran (THF), N-methyl-2-pyrrolidone (NMP), 1,4-dioxane, acetonitrile, or a combination thereof. In some aspects, the polar aprotic solvent is 1,4-dioxane. In some aspects, the inorganic acid is hydrochloric acid. In some aspects, the process of forming a compound of Formula **V,** or a pharmaceutically acceptable salt thereof, occurs at a temperature from about 15 °C to about 25 °C. In some aspects, the process of forming a compound of Formula **V,** or a pharmaceutically acceptable salt thereof, takes about 1 to 2 hours. In some aspects, a pharmaceutically acceptable salt of Formula **V** is isolated. In some aspects, the pharmaceutically acceptable salt of Formula **V** is isolated by filtration. In some aspects, the pharmaceutically acceptable salt of Formula **V** is a hydrochloride salt.

Processes further comprising reacting a compound of Formula **V,** or pharmaceutically acceptable salt of thereof, with an aqueous inorganic acid in a polar protic solvent, under conditions suitable to form Compound **9** 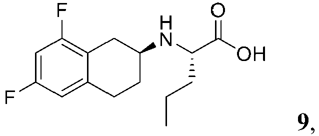 or a pharmaceutically acceptable salt thereof, are also described herein. In some aspects, in the process in which a compound of Formula **V,** or pharmaceutically acceptable salt thereof, is reacted with an aqueous inorganic acid, the polar protic solvent is an alcohol. In some aspects, the alcohol is isopropanol. In some aspects, in the process in which a compound of Formula **V,** or pharmaceutically acceptable salt thereof, is reacted with an aqueous inorganic acid, the inorganic acid is hydrochloric acid. In some aspects, the hydrochloric acid is present in the process in an amount of about 2 to about 6 equivalents of Formula **V.** In some aspects, the hydrochloric acid is present in the process in an amount of about 4.1 equivalents of Formula **V.**

In some aspects, the process of forming Compound **9** occurs at a temperature from about 50 °C to about 70 °C. In some aspects, the process of forming Compound **9** occurs at a temperature from about 58 °C to about 62 °C. In some aspects, the process of forming Compound **9** occurs at a temperature from about 63 °C to about 67 °C. In some aspects, in the process in which a compound of Formula **V,** or pharmaceutically acceptable salt thereof, is reacted with an aqueous inorganic acid, the pH is adjusted to about 0.2 to about 0.6. In some aspects, in the process in which a compound of Formula **V,** or pharmaceutically acceptable salt thereof, is reacted with an aqueous inorganic acid, the pH is adjusted to about 1.2 to about 2.5. In some aspects, in the process in which a compound of Formula **V,** or pharmaceutically acceptable salt thereof, is reacted with an aqueous inorganic acid, the pH is adjusted to about 2.6 to about 3.0. In some aspects, the process of forming Compound **9** occurs in the presence of one or more additives. In some aspects, the one or more additives comprise sodium hydroxide. In some aspects, the process of forming Compound **9** is cooled at a temperature from about 15 °C to about 20 °C. In some aspects, Compound **9,** or pharmaceutically acceptable salt thereof, is isolated. In some aspects, Compound **9,** or pharmaceutically acceptable salt thereof, is isolated by filtration.

The disclosure further relates to processes of preparing Compound **1,** or a pharmaceutically acceptable salt thereof, by reacting Compound **10,** or pharmaceutically acceptable salt thereof, with Compound **11,** or pharmaceutically acceptable salt thereof. In some aspects, the process of preparing Compound **1,** or a pharmaceutically acceptable salt thereof, occurs in the presence of a polar aprotic solvent. In some aspects, the polar aprotic solvent is dimethyl sulfoxide (DMSO), N,N-dimethylformamide (DMF), acetone, tetrahydrofuran (THF), N methyl-2-pyrrolidone (NMP), acetonitrile, or a combination thereof. In some aspects, the polar aprotic solvent is acetonitrile.

Processes comprising reacting Compound **1** free base, prepared by any of the processes described above, with an aqueous inorganic acid under conditions suitable to form a pharmaceutically acceptable salt of Compound **1** are provided herein. In some aspects, the pH of the process of Compound **1** free base with an aqueous inorganic acid is about 1 to about 1.5. In some aspects, the inorganic acid is hydrobromic acid. In some aspects, the pharmaceutically acceptable salt of Compound **1** is isolated. In some aspects, the pharmaceutically acceptable salt of Compound **1** is the hydrobromide salt. In some aspects, the pharmaceutically acceptable salt of Compound **1** is the dihydrobromide salt.

The disclosure further relates to processes comprising reacting Compound **9** in a solvent with 1,1'-carbonyldiimidazole under conditions suitable to form Compound **10,** wherein the process further comprises reacting Compound **10** with Compound **11** under conditions suitable to form Compound **1,** or a pharmaceutically acceptable salt thereof. In some aspects, the process of forming Compound **1,** or a pharmaceutically acceptable salt thereof, occurs in the presence of a polar aprotic solvent. In some aspects, the polar aprotic solvent is dimethyl sulfoxide (DMSO), N,N-dimethylformamide (DMF), acetone, dichloromethane, tetrahydrofuran (THF), N-methyl-2-pyrrolidone (NMP), 1,4-dioxane, acetonitrile, or a combination thereof. In some aspects, the process Compound **1,** or a pharmaceutically acceptable salt thereof, occurs in the presence of one or more additives. In some aspects, the one or more additives comprise pyridine hydrobromide. In some aspects, the one or more additives comprise triethylamine. In some aspects, the process further comprises reacting Compound **1** free base with an aqueous inorganic acid under conditions suitable to form a pharmaceutically acceptable salt of Compound **1.** In some aspects, the pH is adjusted to about 1 to about 1.5. In some aspects, the aqueous inorganic acid is hydrobromic acid.

The disclosure further relates to processes comprising reacting a pharmaceutically acceptable salt of Compound **1** with an aqueous inorganic acid in an alcohol. In some aspects, the alcohol is isopropanol. In some aspects, the aqueous inorganic acid is hydrobromic acid. In some aspects, the hydrobromic acid is present in the process in an amount of about 1 to about 3 equivalents of a pharmaceutically acceptable salt of Compound **1.** In some aspects, the hydrobromic acid is present in the process in an amount of about 2 equivalents of a pharmaceutically acceptable salt of Compound **1.**

In some aspects, the process of forming a pharmaceutically acceptable salt of Compound **1** occurs in the presence of one or more additives. In some aspects, the one or more additives comprise triethylamine. In some aspects, the pH of the process of forming a pharmaceutically acceptable salt of Compound **1** is about 3 to about 3.5.

In some aspects, the process of forming a pharmaceutically acceptable salt of Compound **1** occurs at a temperature from about 40 °C to about 50 °C. In some aspects, the seeds of a pharmaceutically acceptable salt of Compound **1** is added. In some aspects, the amount of the seeds is about 0.5 wt%. In some aspects, the process of forming a pharmaceutically acceptable salt of Compound **1** is cooled to about 5 °C to about 15 °C. In some aspects, a pharmaceutically acceptable salt of Compound **1** is isolated. In some aspects, the pharmaceutically acceptable salt of Compound **1** is isolated as a crystalline solid. In some aspects, the pharmaceutically acceptable salt of Compound **1** is isolated by filtration.

In some aspects, Compound **1** is synthesized by any one of the processes described above. In some aspects, the pharmaceutically acceptable salt of Compound **1** of the processes described herein is isolated. In some aspects, the pharmaceutically acceptable salt of Compound **1** is the hydrobromide salt. In some aspects, the pharmaceutically acceptable salt of Compound **1** is the dihydrobromide salt.

The disclosure further relates to compositions comprising 98.0% to 99.9% of a Compound **1,** or a pharmaceutically acceptable salt thereof, and one or more of the following:
(1) 0.7% to 0.01% of a compound of Formula **II** 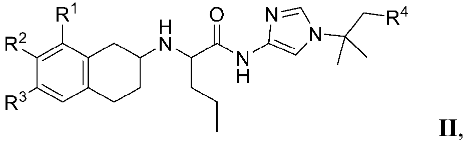 wherein:
   R¹ is selected from the group consisting of hydrogen, fluoro, or chloro;
   R² is selected from the group consisting of hydrogen or chloro;
   R³ is selected from the group consisting of fluoro, or chloro; and
   R⁴ is selected from the group consisting of -OH and -N(H)CH₂C(CH₃)₃,
   or a pharmaceutically acceptable salt thereof;
(2) 0.05% to 0.005% of imidazole, or a pharmaceutically acceptable salt thereof; or
(3) 0.7% to 0.01% of a compound of Formula **II,** or a pharmaceutically acceptable salt thereof, and 0.05% to 0.005% of imidazole, or a pharmaceutically acceptable salt thereof, wherein the compound of Formula **II** is not Compound **1,** and the percentages of Compound **1,** the compound of Formula **II,** and/or imidazole are determined by high performance liquid chromatography. In some aspects, the compound of Formula **II** is a hydrobromide salt. In some aspects, Compound **1** is a hydrobromide salt. In some aspects, Compound **1** is a dihydrobromide salt.

In some aspects, the compound of Formula **II** is a compound of Formula **II-A** 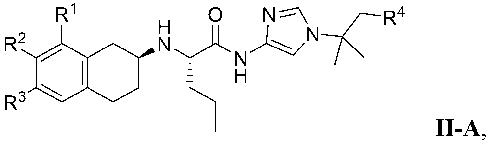 or a pharmaceutically acceptable salt thereof. In some aspects, the compound of Formula **II-A** is a hydrobromide salt. In some aspects, the compound of Formula **II-A** is a dihydrobromide salt.

In some aspects, the compound of Formula **II** is a compound of Formula **II-B** 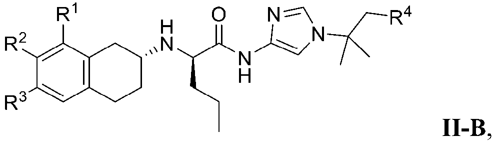 or a pharmaceutically acceptable salt thereof. In some aspects, the compound of Formula **II-B** is a hydrobromide salt. In some aspects, the compound of Formula **II-B** is a dihydrobromide salt.

In some aspects, the compound of Formula **II** is a compound of Formula **II-C** 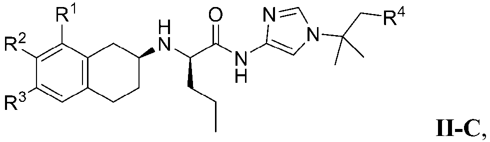 or a pharmaceutically acceptable salt thereof. In some aspects, the compound of Formula **II-C** is a hydrobromide salt. In some aspects, the compound of Formula **II-C** is a dihydrobromide salt.

In some aspects, the compound of Formula **II** is a compound of Formula **II-D** 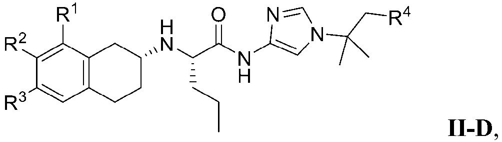 or a pharmaceutically acceptable salt thereof. In some aspects, the compound of Formula **II-D** is a hydrobromide salt. In some aspects, the compound of Formula **II-D** is a dihydrobromide salt.

In some aspects, R⁴ is -N(H)CH₂C(CH₃)₃. In some aspects, R⁴ is -OH. In some aspects, R¹ is fluoro, R² is hydrogen, and R³ is chloro. In some aspects, R¹ is chloro, R² is hydrogen, and R³ is fluoro. In some aspects, R¹ is hydrogen, R² is chloro, and R³ is fluoro. In some aspects, R¹ is fluoro, R² is hydrogen, and R³ is fluoro.

The disclosure further relates to compositions comprising 98.0% to 99.9% of Compound **1,** or a pharmaceutically acceptable salt thereof, and 0.6% to 0.01% of Compound **2** 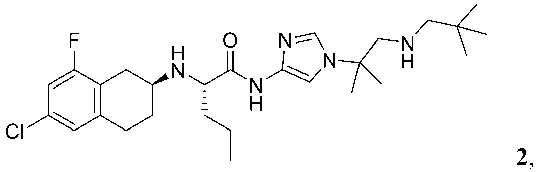 or a pharmaceutically acceptable salt thereof. In some aspects, Compound **2** is a hydrobromide salt. In some aspects, Compound **2** is a dihydrobromide salt. In some aspects, Compound **1** is a hydrobromide salt. In some aspects, Compound **1** is a dihydrobromide salt.

The disclosure further relates to compositions comprising 98.0% to 99.9% of Compound **1,** or a pharmaceutically acceptable salt thereof, and 0.3% to 0.01% of Compound **3** 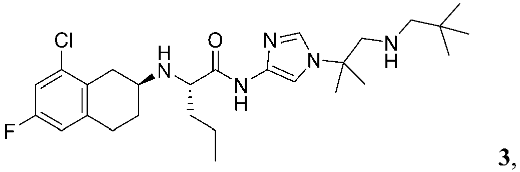 or a pharmaceutically acceptable salt thereof. In some aspects, Compound **3** is a hydrobromide salt. In some aspects, Compound **3** is a dihydrobromide salt. In some aspects, Compound **1** is a hydrobromide salt. In some aspects, Compound **1** is a dihydrobromide salt.

The disclosure further relates to compositions comprising 98.0% to 99.9% of Compound **1,** or a pharmaceutically acceptable salt thereof, and 0.5% to 0.01% of Compound **4** 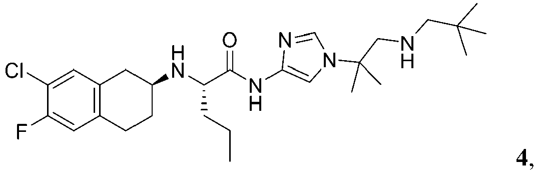 or a pharmaceutically acceptable salt thereof. In some aspects, Compound **4** is a hydrobromide salt. In some aspects, Compound **4** is a dihydrobromide salt. In some aspects, Compound **1** is a hydrobromide salt. In some aspects, Compound **1** is a dihydrobromide salt.

The disclosure further relates to compositions comprising 98.0% to 99.9% of Compound **1,** or a pharmaceutically acceptable salt thereof, and 0.5% to 0.01% of Compound **5** 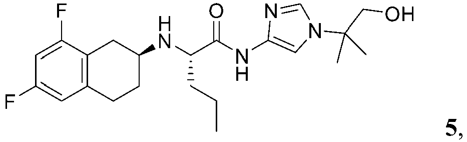 or a pharmaceutically acceptable salt thereof. In some aspects, Compound **5** is a hydrobromide salt. In some aspects, Compound 5 is a dihydrobromide salt. In some aspects, Compound **1** is a hydrobromide salt. In some aspects, Compound **1** is a dihydrobromide salt.

The disclosure further relates to compositions comprising 98.0% to 99.9% of Compound **1,** or a pharmaceutically acceptable salt thereof, and 0.5% to 0.01% of Compound **6** 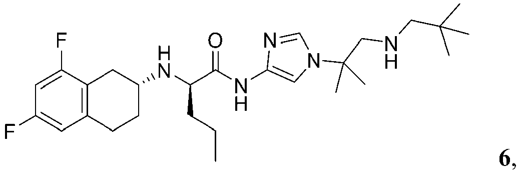 or a pharmaceutically acceptable salt thereof. In some aspects, Compound **6** is a hydrobromide salt. In some aspects, Compound **6** is a dihydrobromide salt. In some aspects, Compound **1** is a hydrobromide salt. In some aspects, Compound **1** is a dihydrobromide salt.

The disclosure further relates to compositions comprising 98.0% to 99.9% of Compound **1,** or a pharmaceutically acceptable salt thereof, and 0.5% to 0.01% of Compound **7** 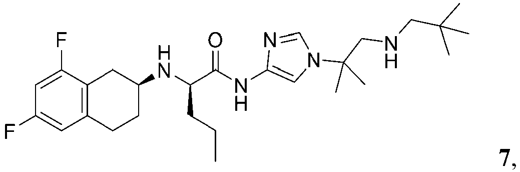 or a pharmaceutically acceptable salt thereof. In some aspects, Compound **7** is a hydrobromide salt. In some aspects, Compound **7** is a dihydrobromide salt. In some aspects, Compound **1** is a hydrobromide salt. In some aspects, Compound **1** is a dihydrobromide salt.

The disclosure further relates to compositions comprising 98.0% to 99.9% of Compound **1,** or a pharmaceutically acceptable salt thereof, and 0.5% to 0.01% of Compound **8** 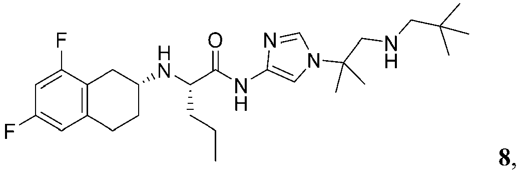 or a pharmaceutically acceptable salt thereof. In some aspects, Compound **8** is a hydrobromide salt. In some aspects, Compound **8** is a dihydrobromide salt. In some aspects, Compound **1** is a hydrobromide salt. In some aspects, Compound **1** is a dihydrobromide salt.

The disclosure further relates to compositions comprising 98.0% to 99.9% of Compound **1,** or a pharmaceutically acceptable salt thereof, and 0.05% to 0.005% of imidazole, or a pharmaceutically acceptable salt thereof. In some aspects, the imidazole is a hydrobromide salt. In some aspects, Compound **1** is a hydrobromide salt. In some aspects, Compound **1** is a dihydrobromide salt.

The disclosure further relates to processes for preparing a composition with an active agent susceptible to oxidation that is substantially free of Compound **13** 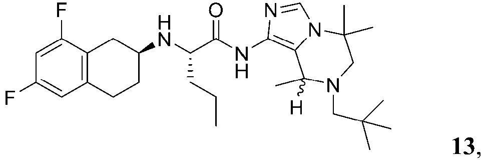 comprising the steps of dissolving Compound **11** in a solvent and combining the solution of Compound **11** with Compound **9** to form a mixture that is treated with hydrobromic acid. In some aspects, the active agent susceptible to oxidation is a hydrobromide salt of Compound **1.** In some aspects, the active agent susceptible to oxidation is a dihydrobromide salt of Compound **1.**

In some aspects, the solvent used to dissolve Compound **11** is an alcohol of the formula R-OH, wherein R is an alkyl, with the proviso that R is not ethyl. In some aspects, the solvent is a primary alcohol, other than ethanol. In some aspects, the solvent is a secondary alcohol. In some aspects, the solvent is a tertiary alcohol. In some aspects, the solvent is 2-methylpropan-1-ol. In some aspects, the solvent is propanol. In some aspects, the solvent is isopropanol. In some aspects, the mixture is warmed to 0-5 °C during hydrobromic acid treatment.

In some aspects, the mixture is maintained at a pH less than 6. In some aspects, the mixture is maintained at a pH less than 3.

The disclosure further relates to processes for preparing a composition of Compound **1,** which is essentially free of Compound **13,** comprising the steps of combining Compound **10** with Compound **11** to form a mixture that is treated with hydrobromic acid in a solvent. In some aspects, the active agent susceptible to oxidation is a hydrobromide salt of Compound **1.** In some aspects, the active agent susceptible to oxidation is a dihydrobromide salt of Compound **1.**

In some aspects, Compound **1** is synthesized by the any one of the processes described above.

### EXAMPLES

The following synthetic examples are illustrative, but not limiting, of the methods described herein. Other suitable modifications and adaptations of the variety of conditions and parameters normally encountered in the field, and which are obvious to those skilled in the art, are within the spirit and scope of the invention.

### SYNTHETIC EXAMPLE 1

Formula V with protecting group *tert*-butyl (1.0 equivalent) is added to isopropanol and water and reacted with aqueous hydrochloric acid (~4.1 equivalents) at approximately 58-62 °C to afford Compound 9. The solution pH is adjusted (~0.2-0.6) with sodium hydroxide, heated to approximately 65 °C, and pH adjusted again to approximately 1.2-2.5. The solution is cooled (~15-20 °C) and adjusted to a final pH of approximately 2.6-3.0 to crystallize the product. Compound 9 is filtered, washed with water and isopropanol, and dried (<75 °C).

### SYNTHETIC EXAMPLE 2

Compound 9 is added to acetonitrile and pyridine hydrobromide (~2.1 equivalents). 1,1'-carbonyldiimidazole (~1.1 equivalents) is added and the mixture is heated (~20-30 °C) to afford Compound 10. Triethylamine (~0.7 equivalents) and acetonitrile are added, and the mixture is cooled (~ -7 °C) and carried forward into next step.

### SYNTHETIC EXAMPLE 3

A dihydrobromide salt of Compound 11 (~1 equivalent) is added to acetonitrile and converted to free base Compound 11 with the addition of triethylamine (~3.1 equivalents) at approximately -7 °C. This solution is carried forward into next step.

### SYNTHETIC EXAMPLE 4

The solution of Compound 11 (~1.20 equivalents) is combined with the solution of Compound 10 (~1 equivalent) at approximately -7 °C to afford a dihydrobromide salt of Compound 1. The reaction mixture is treated with aqueous hydrobromic acid (~7.5 equivalents) and heated to approximately 40 °C. Triethylamine is added to obtain a final mixture pH of about 1 to about 1.5. The seeds of a dihydrobromide salt of Compound 1 (~0.1 wt%) are added, and the mixture is cooled (~5 °C) to crystallize the product. The crude solids of a dihydrobromide salt of Compound 1 are filtered, washed with water and acetonitrile, and dried (~40-50 °C).

### SYNTHETIC EXAMPLE 5

The crude compound of a dihydrobromide salt of Compound 1 (~1 equivalent) is dissolved in isopropanol and water with aqueous hydrobromic acid (~2 equivalents). This solution is heated (about 40 to about 50 °C), neutralized with triethylamine (~1 equivalent), and the seeds of a dihydrobromide salt of Compound 1 (~0. 5 wt%) are added. Triethylamine diluted in isopropanol is added in portions until the target pH (~3-3.5) is reached. The solution is cooled (~5-15 °C) to crystallize the product. The nirogacestat hydrobromide (*e.g.,* nirogacestat dihydrobromide) solids are filtered, washed with pre-cooled (~8-12 °C) isopropanol, and dried (≤65 °C).

### SYNTHETIC EXAMPLE 6

A dry and clean reactor was evacuated and then filled with nitrogen. Methyl *tert-*butyl ether (MTBE) and purified water were charged into the reactor at about 15 to about 25 °C. The mixture was bubbled nitrogen under the surface to degas. At 15~25 °C, hydrogen phosphate salt of Compound 12 (~1 equivalent) was added into the mixture through a solid addition funnel, and stirred for about 0.5 to about 1 hr. At about 15 to about 25 °C, a sodium hydroxide solution (~2 equivalents) was added into the mixture. The mixture was sampled for pH until pH was ≥11. The mixture was filtered. The filter cake was rinsed with MTBE. The filtrate was settled for about 0.5 to about 2 hrs, then separated into an organic phase and an aqueous phase. The aqueous phase was washed with MTBE twice, each time stirred for about 0.5 to about 1 hr and settled for about 0.5 to about 2 hrs, then separated into an organic phase and an aqueous phase. The organic phase was collected and washed with purified water at about 15 to about 25 °C. Then the organic phase was concentrated at a temperature ≤ 40 °C under reduced pressure until about 30 to about 45 L was left. The mixture was sampled for Karl Fischer (KF) analysis (KF≤0.1%). The free base of Compound **12** was synthesized.

Dichloromethane (DCM) was added into the reactor at about 15 to about 25 °C. The mixture was stirred for about 20 to about 30 min.

DCM was added into a second reactor at about 15 to about 25 °C, the mixture was sampled for KF analysis (KF≤0.1%). N,N-Diisopropylethylamine (about 3.4 equivalents) and *tert-butyl* (R)-2-hydroxypentanoate (about 1.7 equivalents) were added into the second reactor at about 15 to about 25 °C, and then the mixture was stirred for about 10 to about 20 min. The mixture was cooled to about -20 to about -30 °C.

DCM was added into a third reactor at about 15 to about 25 °C, the mixture was sampled for KF analysis (KF≤0.1%). Trifluoromethansulfonic anhydride (about 2 equivalents) was added into the third reactor at about 15 to about 25 °C by pump, and then the mixture was stirred for about 10 to about 20 min. Then, the prepared DCM solution with trifluoromethansulfonic anhydride (trifluoromethansulfonic anhydride: DCM=1:6.4 equivalents) was added into the second reactor at -20~-30 °C under stirring. The mixture was allowed to react at about -20 to about - 30 °C. After about 0.5 to about 1 hr, the mixture was sampled every about 1 to about 2 hrs for HPLC analysis, until area% of *tert*-butyl (R)-2-hydroxypentanoate was ≤3.0 area%. A compound of Formula IV was synthesized, with a leaving group as trifluoromethanesulfonate and a protecting group as *tert*-butyl.

The solution of the free base of Compound **12** was added into the solution of the compound of Formula IV at a rate of about 100 to about 150 Kg/h at about -20 to about - 30 °C. The mixture was heated to about 20 to about 30 °C. After about 10 hrs, the mixture was sampled about every 4 to 6 hrs for HPLC analysis until the area% of the free base of Compound **12** was ≤2.0 area% or the difference between two consecutive samples was ≤0.5%. Potassium bicarbonate solution (about 5.0 equivalents) was added into the mixture at about 20 to about 30 °C and the mixture was stirred for about 0.5 to about 1 hr. The mixture was transferred into a stainless steel reactor. The mixture was stirred for about 0.5 to about 1 hr at about 20 to about 30 °C, settled for about 0.5 to about 1 hr, then separated into an organic phase and an aqueous phase. The organic phase was concentrated at a temperature ≤ 40 °C under reduced pressure until about 60 to about 90 L was left. 1,4-dioxane (about 10.0 equivalents) was added into the organic phase at a temperature ≤ 40 °C. The mixture was concentrated at a temperature ≤ 40 °C under reduced pressure until about 60 to about 90 L was left. The mixture was sampled for DCM residual analysis until DCM residual was ≤ 1%. Additional 1,4-dioxane (about 10.0 equivalents) was added into the mixture at about 15 to about 25 °C and stirred for about 0.5 to about 1 hr. The mixture was then filtered. The filter cake was rinsed with 1,4-dioxane. The filtrate was transferred into a reactor at about 15 to about 25 °C and then the mixture was stirred for about 10 to about 20 min. Purified water (about 6.0 equivalents) was added to the mixture at about 15 to about 25 °C. The mixture was adjusted until KF was about 1 to about 2% and then stirred for about 10 to about 20 min. 4M HCl/dioxane solution (about 2 equivalents of HCl) was added to the mixture at about 15 to about 25 °C. The mixture was stirred at about 15 to about 25 °C for crystallization. After about 1 to about 2 hrs, the mixture was sampled for HPLC analysis until the mother liquid of the compound of Formula V with a *tert*-butyl protecting group was ≤0.8wt% or the difference between two consecutive samples was ≤ 0.3%. The slurry in the mixture was filtered. The filter cake was rinsed with 1,4-dioxane three times and then the filter cake was rinsed twice with n-heptane. The solid was dried at a temperature about 40~50 °C. 10 hrs later, the solid was sampled every 3~6 hrs until 1,4-dioxane residual is ≤ 0.5% and n-heptane residual is ≤ 0.5%. After drying, the solid was cooled to 15~25 °C. The hydrochloride salt of a compound of Formula V was synthesized, with a protecting group as *tert*-butyl.

### SYNTHETIC EXAMPLE 7

To obtain Compound 1, Compound 9 may be added to acetonitrile and pyridine hydrobromide (~2.1 equivalents). 1,1'-carbonyldiimidazole (~1.07 equivalents) may then be added and the mixture heated (~20-30 °C) to afford Compound 10. Triethylamine (~0.66 equivalents) and acetonitrile is then added, and the mixture cooled (~ -7 °C). The mixture is then combined with a solution of Compound 11 (1.20 equivalents) at approximately -7 °C to afford Compound 1. The reaction mixture is then treated with aqueous hydrobromic acid (~7.5 equivalents) and heated to approximately 40 °C. Triethylamine may be added to obtain a final mixture pH of approximately 1-1.5. Compound 1 (~0.1 wt%) seeds can be added, and the mixture cooled (~5 °C) to crystallize the product. The crude solid Compound 1 is then filtered, washed with water and acetonitrile, and dried (~40-50 °C). The crude Compound 1 (1.0 equivalent) may then be dissolved in isopropanol and water with aqueous hydrobromic acid (2.0 equivalents). This solution can be heated (~40-50 °C), neutralized with triethylamine (1.0 equivalent), and seeds of Compound 1 (~0. 5 wt%) again added. Triethylamine diluted in isopropanol is then added in portions until the target pH (~3-3.5) is reached. The solution is cooled (~5-15 °C) to crystallize the product. Compound 1 is then filtered, washed with pre-cooled (~8-12 °C) isopropanol, and dried (≤65 °C).

### EMBODIMENTS

1. A process comprising reacting Compound **9** 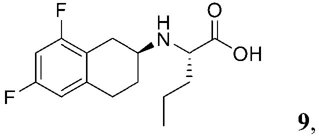 in a solvent with 1,1'-carbonyldiimidazole under conditions suitable to form Compound **10** 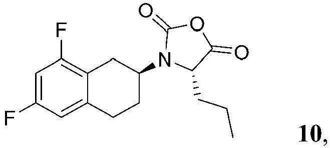 or a pharmaceutically acceptable salt thereof.
2. The process of embodiment 1, wherein the process occurs in the presence of a polar aprotic solvent.
3. The process of embodiment 2, wherein the polar aprotic solvent is dimethyl sulfoxide (DMSO), N,N-dimethylformamide (DMF), acetone, dichloromethane, tetrahydrofuran (THF), N-methyl-2-pyrrolidone (NMP), 1,4-dioxane, acetonitrile, or a combination thereof.
4. The process of any one of embodiments 1-3, wherein the process occurs in the presence of one or more additives.
5. The process of embodiment 4, wherein the one or more additives comprise pyridine hydrobromide.
6. The process of embodiments 4 or 5, wherein the one or more additives comprise triethylamine.
7. The process of any one of embodiments 1-6, wherein Compound **10,** or a pharmaceutically acceptable salt thereof, is used in a subsequent process without isolation or purification.
8. The process of any one of embodiments 1-7, wherein the process further comprises reacting Compound **10** with Compound 11 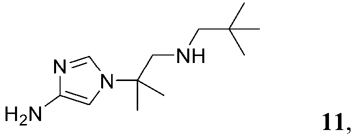 under conditions suitable to form Compound **1** 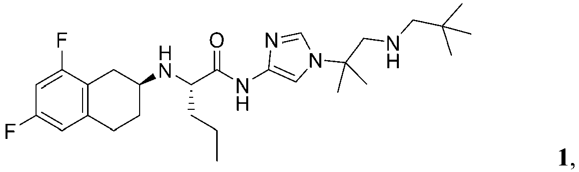 or a pharmaceutically acceptable salt thereof.
9. The process of embodiment 8, wherein the process occurs in the presence of a polar aprotic solvent.
10. The process of embodiment 9, wherein the polar aprotic solvent is dimethyl sulfoxide (DMSO), N,N-dimethylformamide (DMF), acetone, tetrahydrofuran (THF), N-methyl-2-pyrrolidone (NMP), acetonitrile, or a combination thereof.
11. The process of any one of embodiments 8-10, wherein Compound **11** is prepared without isolation or purification, and reacted with Compound **10.**
12. The process of any one of embodiments 8-11, wherein Compound **1** is the free base.
13. The process of any one of embodiments 8-12, wherein Compound **1** is neither purified nor isolated.
14. The process of any one of embodiments 8-11, wherein Compound **1** is a pharmaceutically acceptable salt.
15. The process of embodiment 14, wherein Compound **1** is the dihydrobromide salt.
16. The process of embodiment 15, wherein the dihydrobromide salt of Compound **1** is a crystalline solid.
17. The process of any one of embodiments 12-13, wherein the process further comprises reacting Compound 1 free base with an aqueous inorganic acid, 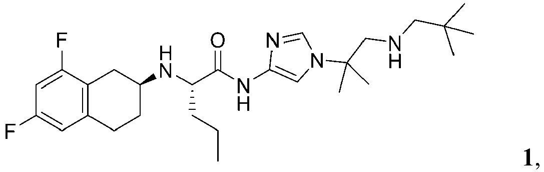 under conditions suitable to form a pharmaceutically acceptable salt of Compound **1.**
18. The process of embodiments 17, wherein the pH is adjusted to about 1 to about 1.5.
19. The process of any one of embodiments 17-18, wherein the inorganic acid is hydrobromic acid.
20. The process of any one of embodiments 17-19, further comprising isolating the pharmaceutically acceptable salt of Compound **1.**
21. The process of embodiment 20, wherein the pharmaceutically acceptable salt of Compound **1** is the hydrobromide salt.
22. The process of embodiment 21, wherein the pharmaceutically acceptable salt of Compound **1** is the dihydrobromide salt.
23. A process of preparing a compound of Formula **V** 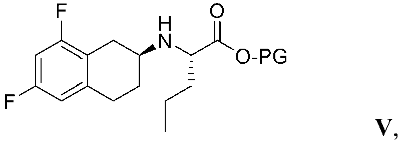 or a pharmaceutically acceptable salt thereof, the process comprising reacting Compound **12** 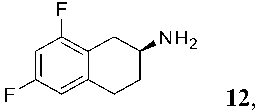 with a compound of Formula **IV** 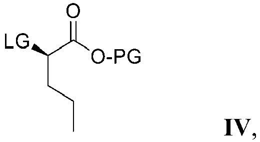 wherein:
   LG is a leaving group; and
   PG is a protecting group,
   under conditions suitable to form a compound of Formula **V,** or a pharmaceutically acceptable salt thereof.
24. The process of embodiment 23, wherein:
   LG is -OR²;
   R² is -S(=O)₂R³; and
   R³ is C₁-C₃ alkyl, C₁-C₃ haloalkyl, or optionally substituted phenyl.
25. The process of embodiment 24, wherein R² is -S(=O)₂CF₃.
26. The process of any one of embodiments 23-25, wherein the process occurs in the presence of a polar aprotic solvent.
27. The process of embodiment 26, wherein the polar aprotic solvent is dimethyl sulfoxide (DMSO), N,N-dimethylformamide (DMF), acetone, dichloromethane, tetrahydrofuran (THF), N-methyl-2-pyrrolidone (NMP), 1,4-dioxane, acetonitrile, or a combination thereof.
28. The process of any one of embodiments 23-27, wherein process occurs in the presence of a base.
29. The process of embodiment 28, wherein the base is N,N-diisopropylethylamine.
30. The process of any one of embodiments 26-29, wherein the solvent is removed *in vacuo.*
31. The process of embodiment 30, wherein the compound of Formula **V** is dissolved in a polar aprotic solvent and an aqueous inorganic acid.
32. The process of embodiment 31, wherein the polar aprotic solvent is dimethyl sulfoxide (DMSO), N,N-dimethylformamide (DMF), acetone, dichloromethane, tetrahydrofuran (THF), N-methyl-2-pyrrolidone (NMP), 1,4-dioxane, acetonitrile, or a combination thereof.
33. The process of embodiments 31 or 32 further comprising isolating a pharmaceutically acceptable salt of Formula **V.**
34. The process of embodiment 33, wherein the pharmaceutically acceptable salt of Formula V is isolated by filtration.
35. The process of any one of embodiments 31-34, wherein the inorganic acid is hydrochloric acid.
36. The process of any one of embodiments 33-35, wherein the pharmaceutically acceptable salt of Formula **V** is the hydrochloride salt.
37. The process of any one of embodiments 23-36, wherein PG is a C₁-C₆ alkyl.
38. The process of embodiment 37, wherein PG is t-butyl.
39. The process of any one of embodiments 37-38, wherein the process further comprises reacting a compound of Formula **V,** or pharmaceutically acceptable salt of thereof, with an aqueous inorganic acid in a polar protic solvent, under conditions suitable to form Compound **9** 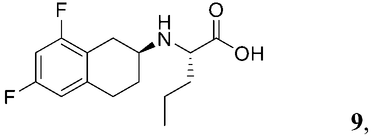 or a pharmaceutically acceptable salt thereof.
40. The process of embodiment 39, wherein the polar protic solvent is an alcohol.
41. The process of embodiment 40, wherein the alcohol is isopropanol.
42. The process of embodiments 39-41, wherein the pH is adjusted to about 2.6 to about 3.0.
43. The process of embodiment 42 further comprising isolating Compound **9,** or pharmaceutically acceptable salt thereof.
44. The process of embodiment 43, wherein Compound **9,** or pharmaceutically acceptable salt thereof, is isolated by filtration.
45. The process of any one of embodiments 39-44, wherein the inorganic acid is hydrochloric acid.
46. A process of preparing Compound 1: 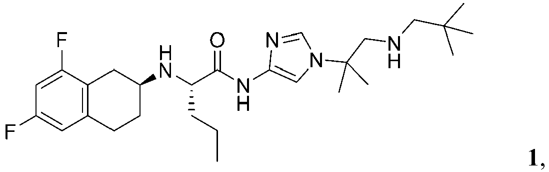 or a pharmaceutically acceptable salt thereof, the process comprising reacting Compound **10,** or pharmaceutically acceptable salt thereof, with Compound **11,** or pharmaceutically acceptable salt thereof, under conditions suitable to form Compound **1,** or a pharmaceutically acceptable salt thereof.
47. The process of embodiment 46, wherein the process occurs in the presence of a polar aprotic solvent.
48. The process of embodiment 47, wherein the polar aprotic solvent is dimethyl sulfoxide (DMSO), N,N-dimethylformamide (DMF), acetone, tetrahydrofuran (THF), N-methyl-2-pyrrolidone (NMP), acetonitrile, or a combination thereof.
49. The process of any one of embodiments 46-48, wherein Compound **11** is prepared without isolation or purification, and reacted with Compound **10.**
50. The process of any one of embodiments 46-49, wherein Compound **1** is the free base.
51. The process of any one of embodiments 46-50, wherein Compound **1** is neither purified nor isolated.
52. The process of any one of embodiments 46-49, wherein Compound **1** is a pharmaceutically acceptable salt.
53. The process of embodiment 52, wherein Compound **1** is the hydrobromide salt.
54. The process of embodiment 53, wherein the hydrobromide salt of Compound **1** is isolated as a crystalline solid.
55. The process of any one of embodiments 50-51, wherein the process further comprises reacting Compound **1** free base with an aqueous inorganic acid, 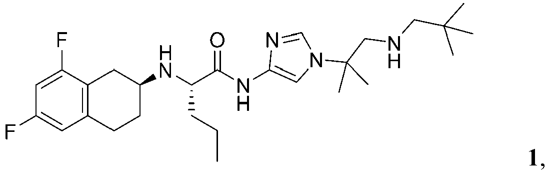 under conditions suitable to form a pharmaceutically acceptable salt of Compound **1.**
56. The process of embodiment 55, wherein the pH is about 1 to about 1.5.
57. The process of any one of embodiments 55-56, wherein the inorganic acid is hydrobromic acid.
58. The process of any one of embodiments 55-57, further comprising isolating the pharmaceutically acceptable salt of Compound **1.**
59. The process of embodiment 58, wherein the pharmaceutically acceptable salt of Compound **1** is the hydrobromide salt.
60. The process of embodiment 59, wherein the pharmaceutically acceptable salt of Compound **1** is the dihydrobromide salt.
61. A process comprising reacting Compound **9** 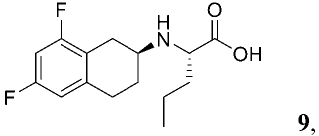 in a solvent with 1,1'-carbonyldiimidazole under conditions suitable to form Compound **10** 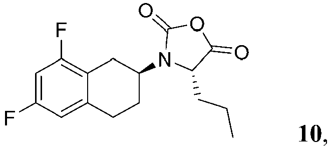 wherein the process further comprises reacting Compound **10** with Compound **11** 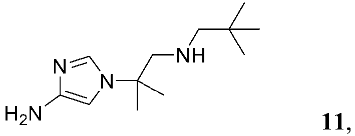 under conditions suitable to form Compound **1** 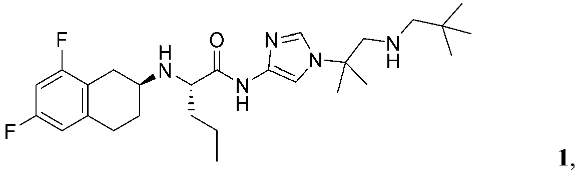 or a pharmaceutically acceptable salt thereof.
62. The process of embodiment 61, the process occurs in the presence of a polar aprotic solvent.
63. The process of embodiment 62, wherein the polar aprotic solvent is dimethyl sulfoxide (DMSO), N,N-dimethylformamide (DMF), acetone, dichloromethane, tetrahydrofuran (THF), N-methyl-2-pyrrolidone (NMP), 1,4-dioxane, acetonitrile, or a combination thereof.
64. The process of any one of embodiments 61-63, wherein the process occurs in the presence of one or more additives.
65. The process of embodiment 64, wherein the one or more additives comprise pyridine hydrobromide.
66. The process of embodiments 64 or 65, wherein the one or more additives comprise triethylamine.
67. The process of any one of embodiments 61-66, wherein Compound **10** is used in a subsequent process without isolation or purification.
68. The process of any one of embodiments 61-67, wherein Compound **11** is prepared without isolation or purification, and reacted with Compound **10.**
69. The process of any one of embodiments 61-68, wherein Compound **1** is the free base.
70. The process of any one of embodiments 61-69, wherein Compound **1** is neither purified nor isolated.
71. The process of any one of embodiments 61-68, wherein Compound **1** is a pharmaceutically acceptable salt.
72. The process of embodiment 71, wherein Compound **1** is the dihydrobromide salt.
73. The process of embodiment 72, wherein the dihydrobromide salt of Compound **1** is isolated as a crystalline solid.
74. The process of any one of embodiments 69-70, wherein the process further comprises reacting Compound **1** free base with an aqueous inorganic acid, 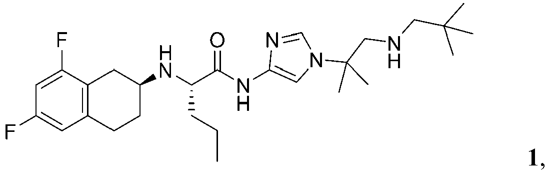 under conditions suitable to form a pharmaceutically acceptable salt of Compound **1.**
75. The process of embodiment 74, wherein the pH is adjusted to about 1 to about 1.5.
76. The process of any one of embodiments 74-75, wherein the aqueous inorganic acid is hydrobromic acid.
77. The process of any one of embodiments 74-76, further comprising isolating the pharmaceutically acceptable salt of Compound **1.**
78. The process of embodiment 77, wherein the pharmaceutically acceptable salt of Compound **1** is the hydrobromide salt.
79. The process of embodiment 78, wherein the pharmaceutically acceptable salt of Compound **1** is the dihydrobromide salt.
80. A process comprising reacting a pharmaceutically acceptable salt of Compound **1:** 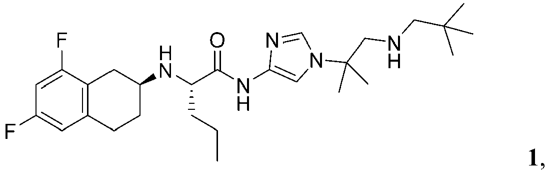 with an aqueous inorganic acid in an alcohol.
81. The process of embodiment 80, wherein the alcohol is isopropanol.
82. The process of any one of embodiments 80-81, wherein the pH is adjusted to about 3 to about 3.5.
83. The process of any one of embodiments 80-82 further comprising isolating a pharmaceutically acceptable salt of Compound **1.**
84. The process of embodiment 83, wherein the pharmaceutically acceptable salt of Compound **1** is isolated by filtration.
85. The process of embodiments any one of embodiments 80-84, wherein the aqueous inorganic acid is hydrobromic acid.
86. The process of any one of embodiments 80-85, wherein the pharmaceutically acceptable salt of Compound **1** is a hydrobromide salt.
87. The process of embodiment 86, wherein the pharmaceutically acceptable salt of Compound **1** is a dihydrobromide salt.
88. A composition comprising: 98.0% to 99.9% of a Compound **1:** 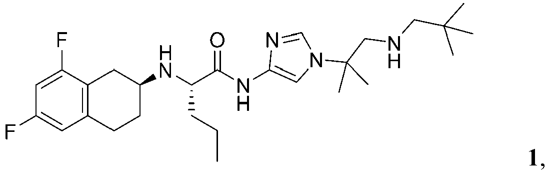 or a pharmaceutically acceptable salt thereof; and one or more of the following:
   (1) 0.7% to 0.01% of a compound of Formula **II:** 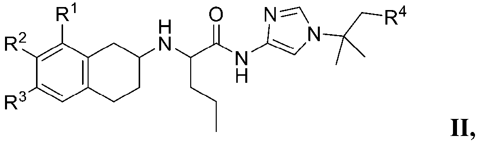 wherein:
      R¹ is selected from the group consisting of hydrogen, fluoro, or chloro;
      R² is selected from the group consisting of hydrogen or chloro;
      R³ is selected from the group consisting of fluoro, or chloro; and
      R⁴ is selected from the group consisting of -OH and -N(H)CH₂C(CH₃)₃,
      or a pharmaceutically acceptable salt thereof;
   (2) 0.05% to 0.005% of imidazole, or a pharmaceutically acceptable salt thereof; or
   (3) 0.7% to 0.01% of a compound of Formula **II,** or a pharmaceutically acceptable salt thereof, and 0.05% to 0.005% of imidazole, or a pharmaceutically acceptable salt thereof,
   wherein the compound of Formula **II** is not Compound **1,** and the percentages of Compound 1, the compound of Formula **II,** and/or imidazole are determined by high performance liquid chromatography.
89. The composition of embodiment 88, wherein the compound of Formula **II** is a hydrobromide salt.
90. The composition of embodiment 88, wherein the compound of Formula **II** is a compound of Formula **II-A:** 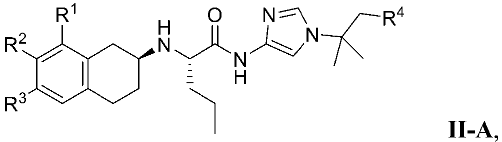 or a pharmaceutically acceptable salt thereof.
91. The composition of embodiment 90, wherein the compound of Formula **II-A** is a hydrobromide salt.
92. The composition of embodiment 88, wherein the compound of Formula **II** is a compound of Formula **II-B:** 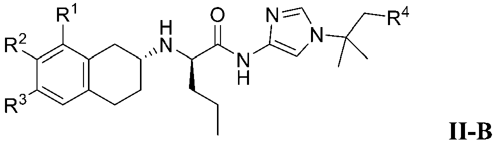 or a pharmaceutically acceptable salt thereof.
93. The composition of embodiment 92, wherein the compound of Formula **II-B** is a hydrobromide salt.
94. The composition of embodiment 88, wherein the compound of Formula **II** is a compound of Formula **II-C:** 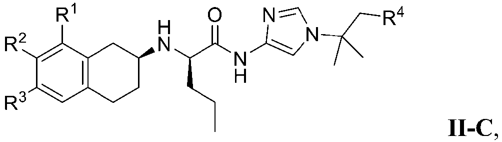 or a pharmaceutically acceptable salt thereof.
95. The composition of embodiment 94, wherein the compound of Formula **II-C** is a hydrobromide salt.
96. The composition of embodiment 88, wherein the compound of Formula **II** is a compound of Formula **II-D:** 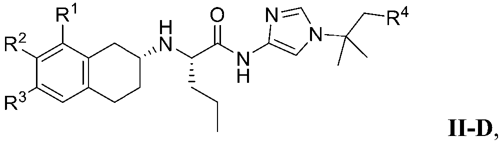 or a pharmaceutically acceptable salt thereof.
97. The composition of embodiment 96, wherein the compound of Formula **II-D** is a hydrobromide salt.
98. The composition of any one of embodiments 88-97, wherein R⁴ is - N(H)CH₂C(CH₃)₃.
99. The composition of any one of embodiments 88-97, wherein R⁴ is -OH.
100. The composition of any one of embodiments 88-99, wherein R¹ is fluoro; R² is hydrogen; and R³ is chloro.
101. The composition of any one of embodiments 88-99, wherein R¹ is chloro; R² is hydrogen; and R³ is fluoro.
102. The composition of any one of embodiments 88-99, wherein R¹ is hydrogen; R² is chloro; and R³ is fluoro.
103. The composition of any one of embodiments 88-99, wherein R¹ is fluoro, R² is hydrogen, and R³ is fluoro.
104. The composition of embodiment 88 comprising:
   (1) 98.0% to 99.9% of Compound **1,** or a pharmaceutically acceptable salt thereof; and
   (2) 0.6% to 0.01% of Compound **2:** 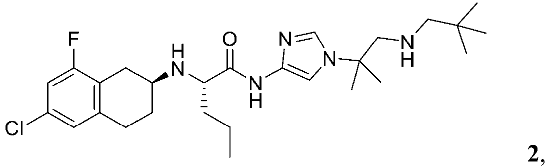 or a pharmaceutically acceptable salt thereof.
105. The composition of embodiment 104, wherein Compound **2** is a dihydrobromide salt.
106. The composition embodiment 88 comprising:
   (1) 98.0% to 99.9% of Compound **1,** or a pharmaceutically acceptable salt thereof; and
   (2) 0.3% to 0.01% of Compound **3:** 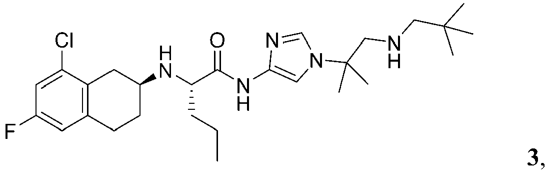 or a pharmaceutically acceptable salt thereof.
107. The composition of embodiment 106, wherein Compound **3** is a dihydrobromide salt.
108. The composition of embodiment 88 comprising:
   (1) 98.0% to 99.9% of Compound **1,** or a pharmaceutically acceptable salt thereof; and
   (2) 0.5% to 0.01% of Compound **4:** 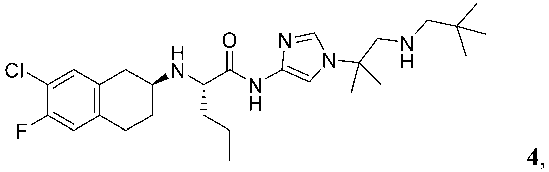 or a pharmaceutically acceptable salt thereof.
109. The composition of embodiment 108, wherein Compound **4** is a dihydrobromide salt.
110. The composition of embodiment 88 comprising:
   (1) 98.0% to 99.9% of Compound **1,** or a pharmaceutically acceptable salt thereof; and
   (2) 0.5% to 0.01% of Compound **5:** 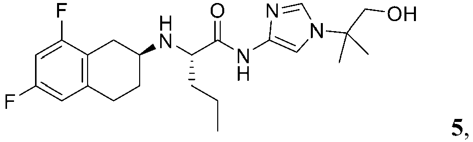 or a pharmaceutically acceptable salt thereof.
111. The composition of embodiment 110, wherein Compound **5** is a dihydrobromide salt.
112. The composition of embodiment 88 comprising:
   (1) 98.0% to 99.9% of Compound **1,** or a pharmaceutically acceptable salt thereof; and
   (2) 0.5% to 0.01% of Compound **6:** 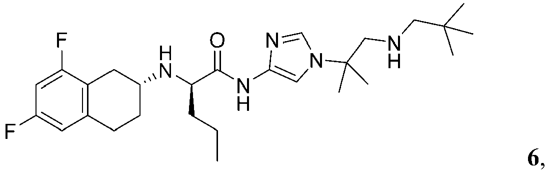 or a pharmaceutically acceptable salt thereof.
113. The composition of embodiment 112, wherein Compound **6** is a dihydrobromide salt.
114. The composition of embodiment 88 comprising:
   (1) 98.0% to 99.9% of Compound **1,** or a pharmaceutically acceptable salt thereof; and
   (2) 0.5% to 0.01% of Compound **7:** 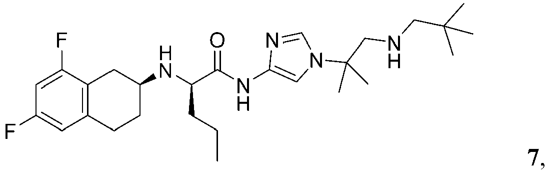 or a pharmaceutically acceptable salt thereof.
115. The composition of embodiment 114, wherein Compound **7** is a dihydrobromide salt.
116. The composition of embodiment 88 comprising:
   (1) 98.0% to 99.9% of Compound **1,** or a pharmaceutically acceptable salt thereof; and
   (2) 0.5% to 0.01% of Compound **8:** 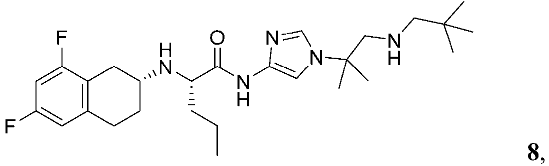 or a pharmaceutically acceptable salt thereof.
117. The composition of embodiment 116, wherein Compound **8** is a dihydrobromide salt.
118. The composition of embodiment 88 comprising:
   (1) 98.0% to 99.9% of Compound **1,** or a pharmaceutically acceptable salt thereof; and
   (2) 0.05% to 0.005% of imidazole, or a pharmaceutically acceptable salt thereof.
119. The composition of embodiment 118, wherein imidazole is a hydrobromide salt.
120. The composition of any one of embodiments 88-119, wherein Compound **1** is a hydrobromide salt.
121. The composition of embodiment 120, wherein Compound **1** is a dihydrobromide salt.
122. A process for preparing a composition with an active agent susceptible to oxidation that is substantially free of Compound **13** 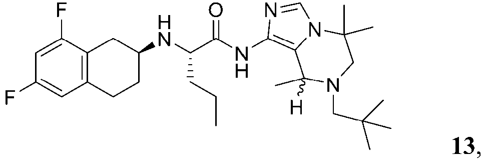 comprising the steps of dissolving Compound **11** in a solvent, 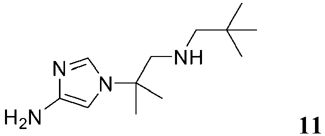 and combining with Compound **9** to form a mixture that is treated with hydrobromic acid, 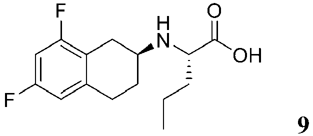 wherein the active agent is a dihydrobromide salt of Compound **1** 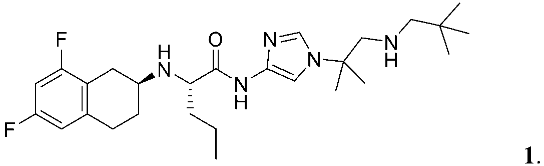
123. A process for preparing a composition which is essentially free of Compound **13** 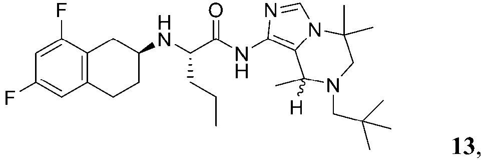 comprising the steps of combining Compound **10** 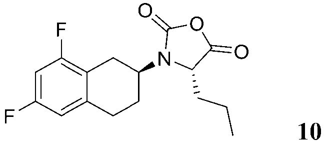 with Compound **11** 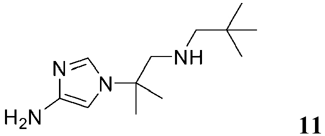 to form a mixture that is treated with hydrobromic acid in a solvent to form an active agent of a dihydrobromide salt of Compound **1** 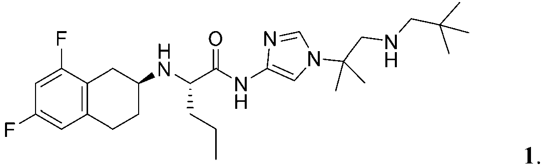
124. The process of embodiment 122, wherein the solvent is an alcohol of the formula R-OH, wherein R is an alkyl, with the proviso that R is not ethyl.
125. The process of embodiment 122, wherein the solvent is a primary alcohol, other than ethanol.
126. The process of embodiment 122, wherein the solvent is a secondary alcohol.
127. The process of embodiment 122, wherein the solvent is a tertiary alcohol.
128. The process of embodiment 122, wherein the solvent is 2-methylpropan-1-ol.
129. The process of embodiment 122, wherein the solvent is propanol.
130. The process of embodiment 122, wherein the solvent is isopropanol.
131. The process of embodiment 122, wherein the mixture is warmed to 0-5 °C during hydrobromic acid treatment.
132. The process of embodiment 122, wherein the mixture is maintained at a pH less than 6.
133. The process of embodiment 122, wherein the mixture is maintained at a pH less than 3.
134. Compound **1** prepared by the process of any one of embodiments 1-22.
135. Compound **1** prepared by the process of any one of embodiments 46-60.
136. Compound **1** prepared by the process of any one of embodiments 61-79.
137. Compound **1** prepared by the process of any one of embodiments 80-87.
138. Compound **1** prepared by the process of any one of embodiments 122 and 124-133.
139. Compound **1** prepared by the process of embodiment 123.

## Claims

1. A composition comprising: 98.0% to 99.9% of a Compound **1:** 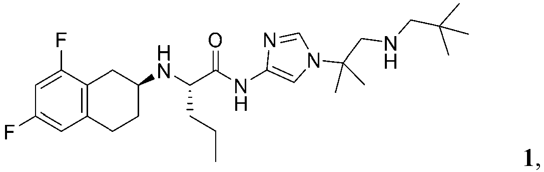 or a pharmaceutically acceptable salt thereof; and one or more of the following:
(1) 0.7% to 0.01% of a compound of Formula **II:** 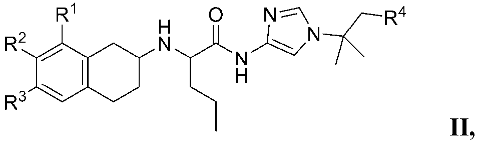 wherein:
R¹ is selected from the group consisting of hydrogen, fluoro and chloro;
R² is selected from the group consisting of hydrogen and chloro;
R³ is selected from the group consisting of fluoro and chloro; and
R⁴ is selected from the group consisting of -OH and -N(H)CH₂C(CH₃)₃,
or a pharmaceutically acceptable salt thereof;
(2) 0.05% to 0.005% of imidazole, or a pharmaceutically acceptable salt thereof; or
(3) 0.7% to 0.01% of a compound of Formula **II,** or a pharmaceutically acceptable salt thereof, and 0.05% to 0.005% of imidazole, or a pharmaceutically acceptable salt thereof, wherein the compound of Formula **II** is not Compound 1, and the percentages of Compound 1, the compound of Formula **II,** and/or imidazole are determined by high performance liquid chromatography, optionally wherein the compound of Formula **II** is a hydrobromide salt.

2. The composition of claim 1, wherein the compound of Formula **II** is a compound of Formula **II-A:** 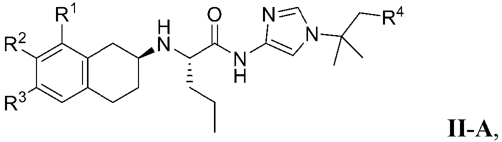 or a pharmaceutically acceptable salt thereof, optionally wherein the compound of Formula **II-A** is a hydrobromide salt.

3. The composition of claim 1, wherein the compound of Formula **II** is a compound of Formula **II-B:** 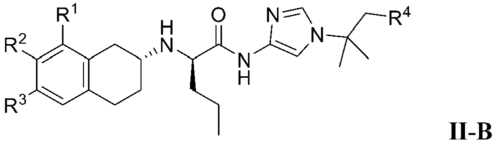 or a pharmaceutically acceptable salt thereof, optionally wherein the compound of Formula **II-B** is a hydrobromide salt.

4. The composition of claim 1, wherein the compound of Formula **II** is a compound of Formula **II-C:** 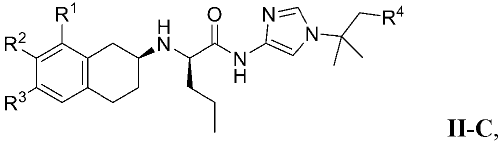 or a pharmaceutically acceptable salt thereof, optionally wherein the compound of Formula **II-C** is a hydrobromide salt.

5. The composition of claim 1, wherein the compound of Formula II is a compound of Formula II-D: 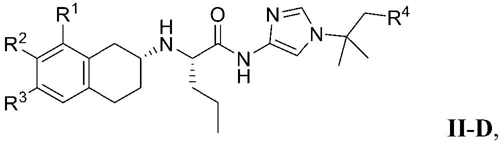 or a pharmaceutically acceptable salt thereof, optionally wherein the compound of Formula II-D is a hydrobromide salt.

6. The composition of any one of claims 1-5, wherein R⁴ is -N(H)CH₂C(CH₃)₃ or -OH, and/or
R¹ is fluoro, R² is hydrogen, and R³ is chloro, or
R¹ is chloro, R² is hydrogen, and R³ is fluoro, or
R¹ is hydrogen; R² is chloro; and R³ is fluoro, or
R¹ is fluoro, R² is hydrogen, and R³ is fluoro.

7. The composition of claim 1, comprising:
(1) 98.0% to 99.9% of Compound **1:** 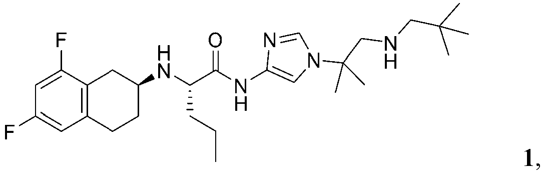 or a pharmaceutically acceptable salt thereof, optionally wherein Compound 1 is a hydrobromide salt; and
(2) 0.6% to 0.01% of Compound **2:** 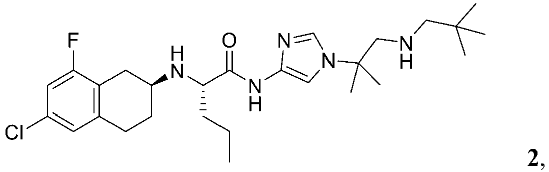 or a pharmaceutically acceptable salt thereof, optionally wherein Compound **2** is a dihydrobromide salt.

8. The composition of claim 1, comprising:
(1) 98.0% to 99.9% of Compound **1:** 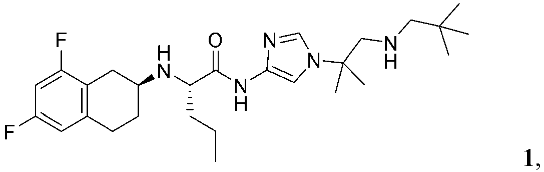 or a pharmaceutically acceptable salt thereof, optionally wherein Compound 1 is a hydrobromide salt; and
(2) 0.3% to 0.01% of Compound **3:** 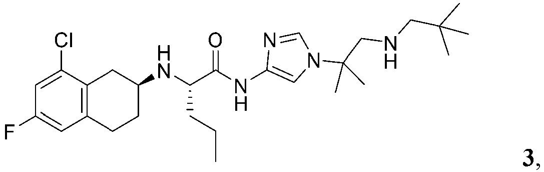 or a pharmaceutically acceptable salt thereof, optionally wherein Compound 3 is a dihydrobromide salt.

9. The composition of claim 1, comprising:
(1) 98.0% to 99.9% of Compound **1:** 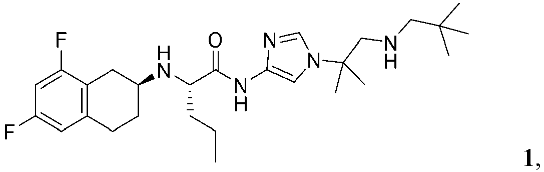 or a pharmaceutically acceptable salt thereof, optionally wherein Compound **1** is a hydrobromide salt; and
(2) 0.5% to 0.01% of Compound **4:** 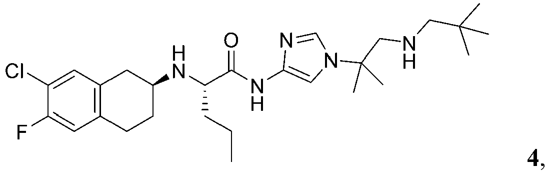 or a pharmaceutically acceptable salt thereof, optionally wherein Compound **4** is a dihydrobromide salt;

10. The composition of claim 1, comprising:
(1) 98.0% to 99.9% of Compound **1:** 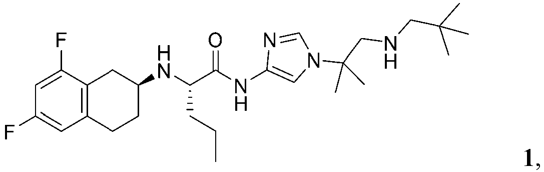 or a pharmaceutically acceptable salt thereof, optionally wherein Compound **1** is a hydrobromide salt; and
(2) 0.5% to 0.01% of Compound **5:** 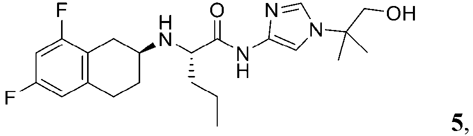 or a pharmaceutically acceptable salt thereof, optionally wherein Compound **5** is a dihydrobromide salt;

11. The composition of claim 1, comprising:
(1) 98.0% to 99.9% of Compound **1:** 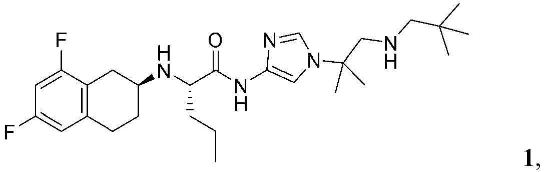 or a pharmaceutically acceptable salt thereof, optionally wherein Compound 1 is a hydrobromide salt; and
(2) 0.5% to 0.01% of Compound **6:** 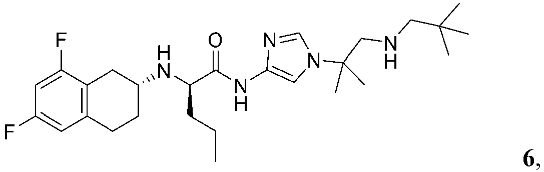 or a pharmaceutically acceptable salt thereof, optionally wherein Compound **6** is a dihydrobromide salt.

12. The composition of claim 1, comprising:
(1) 98.0% to 99.9% of Compound **1:** 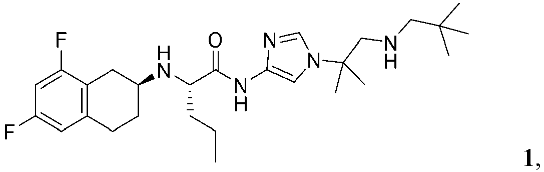 or a pharmaceutically acceptable salt thereof, optionally wherein Compound 1 is a hydrobromide salt; and
(2) 0.5% to 0.01% of Compound **7:** 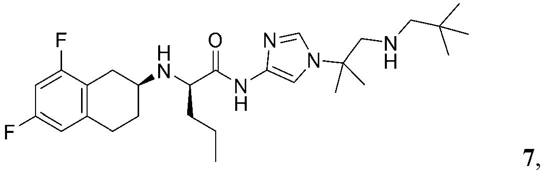 or a pharmaceutically acceptable salt thereof, optionally wherein Compound **7** is a dihydrobromide salt;

13. The composition of claim 1, comprising:
(1) 98.0% to 99.9% of Compound **1:** 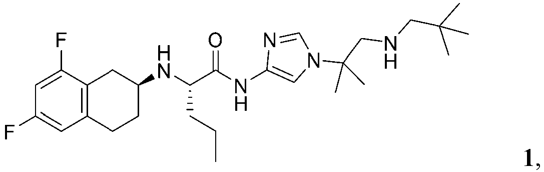 or a pharmaceutically acceptable salt thereof, optionally wherein Compound **1** is a hydrobromide salt; and
(2) 0.5% to 0.01% of Compound **8:** 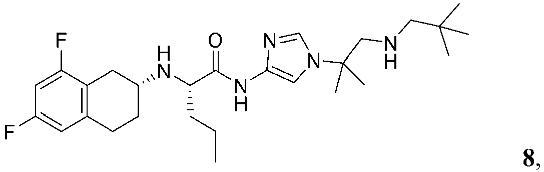 or a pharmaceutically acceptable salt thereof, optionally wherein Compound **8** is a dihydrobromide salt; or

14. The composition of claim 1, comprising:
(1) 98.0% to 99.9% of Compound **1:** 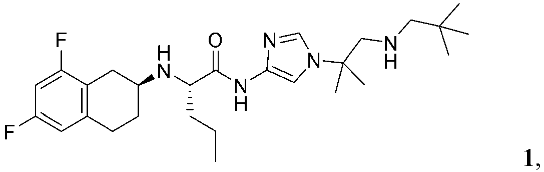 or a pharmaceutically acceptable salt thereof, optionally wherein Compound **1** is a hydrobromide salt; and
(2) 0.05% to 0.005% of imidazole, or a pharmaceutically acceptable salt thereof, optionally wherein the imidazole is a hydrobromide salt.

15. The composition of any one of claims 1-14, wherein Compound **1** is a dihydrobromide salt.
